# EUROPEAN PATENT APPLICATION

(11) **EP 3 769 768 A1**
(43) Date of publication of application: **27.01.2021**
(21) Application number: 19382626.0
(22) Date of filing: 23.07.2019
(51) Int. Cl.: A61K 31/713, A61K 31/416, A61K 31/5377, A61P 25/16

(54) **COMPOUNDS USEFUL FOR THE TREATMENT OF PARKINSON S DISEASE**

(71) Applicant: Consejo Superior de Investigaciones Científicas (CSIC), 28006 Madrid (ES); Université de Lille, 59000 Lille (FR); INSERM (Institut National de la Santé et de la Recherche Médicale), 75013 Paris (FR); Centre Hospitalier Regional Universitaire de Lille, 59037 Lille (FR)
(72) Inventor: NAVARRO HILFIKER, Sabine, 28006 Madrid (ES); FERNÁNDEZ LÓPEZ, María Belén, 28006 Madrid (ES); LARA ORDÓÑEZ, Antonio Jesús, 28006 Madrid (ES); CHARTIER-HARLIN, Marie-Christine, 59139 Wattignies (FR); MUTEZ, Eugénie, 59037 Lille cedex (FR); TAYMANS, Jean-Marc, 1420 Braine-l'Alleud (BE)
(74) Representative: Pons

(57) **Abstract**

The present invention relates to a leucine-rich repeat kinase 2 (LRRK2) inhibitor for use in the treatment and/or prevention of Parkinson's disease, or diseases related to Parkinson's disease, in a subject having centrosomal cohesion deficit, as well as to an *in vitro* method for designing a customized therapy for a subject with Parkinson's Disease, opening a new therapeutic window in the treatment of this disease.

## Description

The invention relates to compounds for use in the treatment and/or prevention of sporadic Parkinson's disease. Thus, the present invention relates to the technical field of the treatment of the neurodegenerative diseases, specifically, to the treatment of Parkinson's disease (PD).

### BACKGROUND ART

The leucine-rich repeat kinase 2 (LRRK2) is a protein involved in a number of intracellular vesicular trafficking events and also plays an important role in neurite outgrowth/cell polarity and cell migration. In dividing cells, pathogenic LRRK2 is known to impair neuronal precursor cell division in vitro and adult neurogenesis in vivo, deficits which may at least in part contribute to some age-dependent non-motor symptoms of Parkinson's disease (PD) patients. LRRK2 is also highly expressed in non-neuronal tissues, suggesting that it may play a more general cellular role(s) shared amongst distinct cell types. Whilst displaying a broad subcellular distribution, LRRK2 can also partially localize to centrosomal compartments.

Mutations in the LRRK2 gene cause autosomal dominant PD, and sequence variations at the LRRK2 locus are associated with increased risk for sporadic PD. The Gly2019Ser (G2019S) mutation in LRRK2 is a relatively common cause of familial Parkinson's disease in Caucasians. It may also cause sporadic Parkinson's disease. The mutated Gly amino acid is conserved in all kinase domains of all species.

The Gly2019Ser mutation is one of a small number of LRRK2 mutations proven to cause Parkinson's disease. Of these, Gly2019Ser is the most common in the Western World, accounting for -2% of all Parkinson's disease cases in North American Caucasians. This mutation is enriched in certain populations, being found in approximately 20% of all Ashkenazi Jewish Parkinson's disease patients and in up to 40% of all Parkinson's disease patients of North African Berber Arab ancestry.

It has been shown that pathogenic LRRK2 causes centrosomal cohesion deficits in human skin fibroblasts from G2019S LRRK2 patients as compared to healthy controls (Madero-Pérez, J. et al., 2018, Molecular Neurodegeneration 13:3). In addition, studies suggest that pathogenic LRRK2 impairs ciliogenesis, another centrosome-related event (Steger, M. et al., 2017, eLIFE, 6:331012; Dhekne, H.S. et al. 2018, eLIFE, 7: e40202). Since pathogenic point mutations in LRRK2 increase its kinase activity, a range of LRRK2 kinase inhibitors have been developed, which are at various stages of preclinical and clinical development. Recent studies indicate that increased kinase activity may also be associated with sporadic Parkinson's disease (DiMaio et al. 2018, Sci. Transl. Med., 10(451): eaar5429). Therefore, LRRK2 kinase inhibitors may also be useful for the treatment of at least a subset of sporadic Parkinson's disease patients, even though no patient stratification methods have been developed.

The international application WO2011057204 discloses inhibitors of LRRK2 kinase that are protective in in vitro and in vivo models of LRRK2-induced neurodegeneration.

### DETAILED DESCRIPTION OF THE INVENTION

The inventors have found that Parkinson's disease (PD) patients having centrosomal deficits can surprisingly benefit from leucine-rich repeat kinase 2 (LRRK2)-related therapeutic interventions. Additionally, the inventors have also found that, in particular, the patients who can benefit from LRRK2 do not comprise the G2019S mutation in the LRRK2 protein (called herewith G2019S LRRK2 mutation), or if the patients suffer from early stages of PD, they comprise the G2019S LRRK2 mutation.

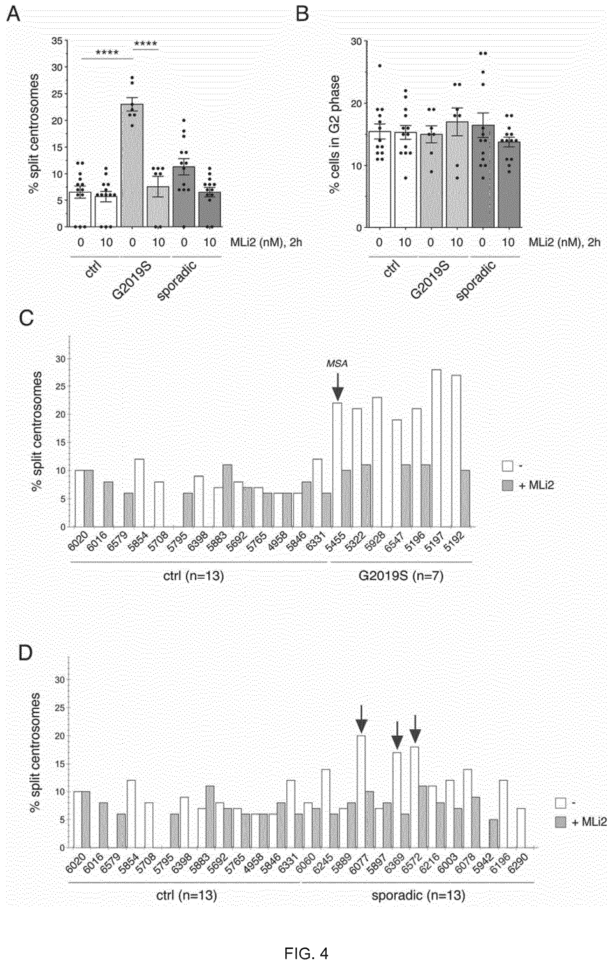

The inventors arrived at this finding by analyzing 13 G2019S LRRK2 PD and 14 sporadic PD samples. In this larger sampling, a significant centrosomal cohesion deficit was observed in lymphoblasts from G2019S LRRK2 PD patients as compared to healthy controls as well, and centrosomal alterations were reverted upon short-term application of 10 nM MLi2 (Figures 4A and 4B). Centrosomal deficits were observed in all G2019S LRRK2 PD samples as compared to control samples, and were reverted by MLi2 in all cases (Figure 4C). Importantly, a significant centrosomal cohesion deficit was also observed in a patient with Multi System Atrophy (MSA) carrying a G2019S mutation and a subset of sporadic PD patients, none of latter were carriers of the G2019S LRRK2 mutation, as compared to healthy controls (Figures 4A and 4B). In three out of 14 sporadic PD patients, centrosomal cohesion deficits were similar to those observed in G2019S LRRK2-PD patients, and were reverted by short-term application of 10 nM MLi2 (Figures 4A to 4D).

In view of the foregoing, the present invention allows the skilled person in the art to identify those PD patients who can benefit from LRRK2-related therapeutic interventions as LRRK2 inhibitors. Hence, the present invention opens a new therapeutic window to those PD patients who show centrosomal deficits but do not carry the G2019S LRRK2 mutation.

Thus, in one aspect, the present invention relates to a leucine-rich repeat kinase 2 (LRRK2) protein inhibitor, hereinafter "inhibitor of the invention", for use in the treatment and/or prevention of Parkinson's disease (PD), or diseases related to PD such as MSA, in a subject comprising (or having or suffering from) centrosomal deficit.

LRRK2 is a protein encoded by the LRRK2 gene. The LRRK2 gene is also known as AURA17, DARDARIN, PARK8, RIPK7 and ROCO2. The transcription of this gene gives rise to nine transcripts, each transcript encoding the different isoforms of the LRRK2 protein as shown in table 1 below.

**Table 1 - Transcripts and proteins corresponding to the gene LRRK2, ENSG00000188906, Human (GRCh38.p12), location Chromosome 12: 40,196,744-40,369,285 forward strand. GRCh38:CM000674.2.**

| **Name** | **Transcript ID Ensembl** | **Isoform of LRRK2 protein UniProtKB ID** |
|---|---|---|
| LRRK2-201 | ENST00000298910.12 | Q5S007 (SEQ ID NO: 1) |
| LRRK2-202 | ENST00000343742.6 | E9PC85 (SEQ ID NO: 2) |
| LRRK2-208 | ENST00000636518.1 | A0A1B0GUQ3 (SEQ ID NO: 3) |
| LRRK2-203 | ENST00000416796.5 | C9JBF0 (SEQ ID NO: 4) |
| LRRK2-204 | ENST00000430804.5 | H7C3B6 (SEQ ID NO: 5) |
| LRRK2-209 | ENST00000644108.1 | A0A2R8Y4F8 (SEQ ID NO: 6) |
| LRRK2-207 | ENST00000481256.1 | No protein |
| LRRK2-206 | ENST00000479187.5 | No protein |
| LRRK2-205 | ENST00000474202.1 | No protein |

This gene is a member of the leucine-rich repeat kinase family and encodes a protein with an armadillo type fold, an ankryin repeat region, a leucine-rich repeat (LRR) domain, a kinase/MLK-like kinase domain, a DFG-like motif, a RAS domain/GTPase domain (termed Ras of complex proteins, or ROC, domain), a C-terminal of ROC (COR) and a WD40 domain. The protein is present largely in the cytoplasm. In a particular embodiment, LRRK2 protein comprises an amino acid sequence having a sequence identity of, at least, 60, 65, 70, 75, 80, 85, 90, 95, 96, 97, 98 or 99 % with the sequence SEQ ID NO: 1 (Unitprot access number Q5S007; NCBI, ACCESSION: NP_940980 XP_058513, XP_935913, XP_935925, XP_941498, XP_947897; VERSION: NP_940980.4; SOURCE: Homo sapiens). In a more particular embodiment, the LRRK2 protein can be any of the isoforms cited in Table 1 (SEQ ID NO: 2 to SEQ ID NO: 6).

SEQ ID NO: 1
MASGSCQGCEEDEETLKKLIVRLNNVQEGKQIETLVQILEDLLVFTYSERASKLFQGKNI
HVPLLIVLDSYMRVASVQQVGWSLLCKLIEVCPGTMQSLMGPQDVGNDWEVLGVHQLILK
MLTVHNASVNLSVIGLKTLDLLLTSGKITLLILDEESDIFMLIFDAMHSFPANDEVQKLG
CKALHVLFERVSEEQLTEFVENKDYMILLSALTNFKDEEEIVLHVLHCLHSLAIPCNNVE
VLMSGNVRCYNIVVEAMKAFPMSERIQEVSCCLLHRLTLGNFFNILVLNEVHEFVVKAVQ
QYPENAALQISALSCLALLTETIFLNQDLEEKNENQENDDEGEEDKLFWLEACYKALTWH
RKNKHVQEAACWALNNLLMYQNSLHEKIGDEDGHFPAHREVMLSMLMHSSSKEVFQASAN
ALSTLLEQNVNFRKILLSKGIHLNVLELMQKHIHSPEVAESGCKMLNHLFEGSNTSLDIM
AAVVPKILTVMKRHETSLPVQLEALRAILHFIVPGMPEESREDTEFHHKLNMVKKQCFKN
DIHKLVLAALNRFIGNPGIQKCGLKVISSIVHFPDALEMLSLEGAMDSVLHTLQMYPDDQ
EIQCLGLSLIGYLITKKNVFIGTGHLLAKILVSSLYRFKDVAEIQTKGFQTILAILKLSA
SFSKLLVHHSFDLVIFHQMSSNIMEQKDQQFLNLCCKCFAKVAMDDYLKNVMLERACDQN
NSIMVECLLLLGADANQAKEGSSLICQVCEKESSPKLVELLLNSGSREQDVRKALTISIG
KGDSQIISLLLRRLALDVANNSICLGGFCIGKVEPSWLGPLFPDKTSNLRKQTNIASTLA
RMVIRYQMKSAVEEGTASGSDGNFSEDVLSKFDEWTFIPDSSMDSVFAQSDDLDSEGSEG
SFLVKKKSNSISVGEFYRDAVLQRCSPNLQRHSNSLGPIFDHEDLLKRKRKILSSDDSLR
SSKLQSHMRHSDSISSLASEREYITSLDLSANELRDIDALSQKCCISVHLEHLEKLELHQ

NALTSFPQQLCETLKSLTHLDLHSNKFTSFPSYLLKMSCIANLDVSRNDIGPSVVLDPTV
KCPTLKQFNLSYNQLSFVPENLTDVVEKLEQLILEGNKISGICSPLRLKELKILNLSKNH
ISSLSENFLEACPKVESFSARMNFLAAMPFLPPSMTILKLSQNKFSCIPEAILNLPHLRS
LDMSSNDIQYLPGPAHWKSLNLRELLFSHNQISILDLSEKAYLWSRVEKLHLSHNKLKEI
PPEIGCLENLTSLDVSYNLELRSFPNEMGKLSKIWDLPLDELHLNFDFKHIGCKAKDIIR
FLQQRLKKAVPYNRMKLMIVGNTGSGKTTLLQQLMKTKKSDLGMQSATVGIDVKDWPIQI
RDKRKRDLVLNVWDFAGREEFYSTHPHFMTQRALYLAVYDLSKGQAEVDAMKPWLFNIKA
RASSSPVILVGTHLDVSDEKQRKACMSKITKELLNKRGFPAIRDYHFVNATEESDALAKL
RKTIINESLNFKIRDQLVVGQLIPDCYVELEKIILSERKNVPIEFPVIDRKRLLQLVREN
QLQLDENELPHAVHFLNESGVLLHFQDPALQLSDLYFVEPKWLCKIMAQILTVKVEGCPK
HPKGIISRRDVEKFLSKKRKFPKNYMSQYFKLLEKFQIALPIGEEYLLVPSSLSDHRPVI
ELPHCENSEIIIRLYEMPYFPMGFWSRLINRLLEISPYMLSGRERALRPNRMYWRQGIYL
NWSPEAYCLVGSEVLDNHPESFLKITVPSCRKGCILLGQVVDHIDSLMEEWFPGLLEIDI
CGEGETLLKKWALYSFNDGEEHQKILLDDLMKKAEEGDLLVNPDQPRLTIPISQIAPDLI
LADLPRNIMLNNDELEFEQAPEFLLGDGSFGSVYRAAYEGEEVAVKIFNKHTSLRLLRQE
LVVLCHLHHPSLISLLAAGIRPRMLVMELASKGSLDRLLQQDKASLTRTLQHRIALHVAD
GLRYLHSAMIIYRDLKPHNVLLFTLYPNAAIIAKIADYGIAQYCCRMGIKTSEGTPGFRA
PEVARGNVIYNQQADVYSFGLLLYDILTTGGRIVEGLKFPNEFDELEIQGKLPDPVKEYG
CAPWPMVEKLIKQCLKENPQERPTSAQVFDILNSAELVCLTRRILLPKNVIVECMVATHH
NSRNASIWLGCGHTDRGQLSFLDLNTEGYTSEEVADSRILCLALVHLPVEKESWIVSGTQ
SGTLLVINTEDGKKRHTLEKMTDSVTCLYCNSFSKQSKQKNFLLVGTADGKLAIFEDKTV
KLKGAAPLKILNIGNVSTPLMCLSESTNSTERNVMWGGCGTKIFSFSNDFTIQKLIETRT
SQLFSYAAFSDSNIITVVVDTALYIAKQNSPVVEVWDKKTEKLCGLIDCVHFLREVMVKE
NKESKHKMSYSGRVKTLCLQKNTALWIGTGGGHILLLDLSTRRLIRVIYNFCNSVRVMMT
AQLGSLKNVMLVLGYNRKNTEGTQKQKEIQSCLTVWDINLPHEVQNLEKHIEVRKELAEK
MRRTSVE

In the present invention, "identity" or "sequence identity" is understood to mean the degree of similarity between two nucleotides or amino acid sequences obtained by aligning the two sequences. Depending on the number of common residues between the aligned sequences, a different degree of identity, expressed as a percentage, will be obtained. The degree of identity between two amino acid sequences may be determined by conventional methods, for example, by standard sequence alignment algorithms known in the state of the art, such as, for example, BLAST [Altschul S.F. et al. Basic local alignment search tool. J Mol Biol. 1990 Oct 5; 215(3): 403-10]. The BLAST programs, for example, BLASTN, BLASTX, and T BLASTX, BLASTP and TBLASTN, are in the public domain at The National Center for Biotechonology Information (NCBI) website. The skilled person in the art understands that mutations in the nucleotide sequence of genes that lead to conservative amino acid substitutions at non-critical positions for the functionality of the protein might be evolutionarily neutral mutations which do not affect its global structure or its functionality.

In another particular embodiment, the LRRK2 protein comprises an amino acid sequence having a sequence identity of, at least, 100% with the sequence SEQ ID NO: 1. In a most particular embodiment, the LRRK2 protein consists of the amino acid sequence SEQ ID NO: 1.

The present invention comprises the use of a LRRK2 protein inhibitor. The terms "LRRK2 protein inhibitor" and "LRRK2 inhibitor" are considered equivalent and can be used distinguishingly throughout the present description. In the present invention, the term "LRRK2 inhibitor" refers to any molecule capable of completely or partially inhibiting the LRRK2 gene expression, both by preventing the expression product of said gene from being produced (interrupting the LRRK2 gene transcription and/or blocking the translation of the mRNA coming from the LRRK2 gene expression) and by directly inhibiting the LRRK2 protein kinase activity. Methods for decreasing/abrogating the expression of the gene encoding the LRRK2 protein include, without being limited to, editing technologies such as CRISPR/cas9 or Cas9 nickase technology.

LRRK2 inhibitors can be identified using methods based on the capacity of the so-called inhibitor to cause dephosphorylation of the N-terminal cellular phosphorylation sites of LRRK2, which is assessed using Western blotting with antibodies against phospho-Ser935 LRRK2, or to cause dephosphorylation of Rab10, a LRRK2 kinase substrate, which is assessed using Western blotting with antibodies against phospho-Thr73 Rab10. Nevertheless, assays to evaluate whether a given compound is a LRRK2 inhibitor can be found, for example, in the patent applications GB2479799, WO/2008/091799, US20110519443, US20070881677, GB20070006709 and US20110519443.

By way of non-limiting illustration, LRRK2 inhibitors suitable for use in the present invention include chemical compounds, antisense oligonucleotides, interference RNAs (siRNAs), catalytic RNAs or specific ribozymes and inhibitory antibodies. Thus, in a particular embodiment, the LRRK2 inhibitor is selected from the group consisting of a chemical compound, a LRRK2 specific siRNA, a LRRK2 specific antisense oligonucleotide, a LRRK2 specific ribozyme, and an inhibitory LRRK2 specific antibody.

### Antisense Oligonucleotides

An additional aspect of the invention relates to the use of isolated "antisense" nucleic acids to inhibit expression, for example, for inhibiting transcription and/or translation of a nucleic acid which encodes LRRK2, the activity of which is to be inhibited. The antisense nucleic acids can be bound to the target potential of the drug by means of conventional base complementarity or, for example, in the case of binding to double stranded DNA through specific interaction in the large groove of the double helix. Generally, these methods refer to a range of techniques generally used in the art and they include any method which is based on the specific binding to oligonucleotide sequences.

An antisense oligonucleotide can be distributed, for example, as an expression plasmid which, when it is transcribed in cell, produces RNA complementary to at least one unique part of the cellular mRNA encoding LRRK2. Alternatively, the antisense oligonucleotide is an oligonucleotide probe generated *ex vivo* which, when introduced into the cell, produces inhibition of gene expression hybridizing with the mRNA and/or gene sequences of a target nucleic acid. Such oligonucleotide probes are preferably modified oligonucleotides which are resistant to endogenous nucleases, for example, exonucleases and/or endonucleases and are therefore stable in vivo. Examples of nucleic acid molecules for use thereof as antisense oligonucleotides are DNA analogs of phosphoramidate, phosphothionate and methylphosphonate.

With respect to the antisense oligonucleotide, the oligodeoxyribonucleotide regions derived from the starting site of the translation, for example, between -10 and +10 of the target gene are preferred. The antisense approximations involve the oligonucleotide design (either DNA or RNA) that are complementary to the mRNA encoding the target polypeptide. The antisense oligonucleotide will be bound to the transcribed mRNA and translation will be prevented.

The oligonucleotides which are complementary to the 5' end of the mRNA, for example the non-translated 5' sequence up to and including the start codon AUG must function in the most efficient manner to inhibit translation. Nevertheless, it has been shown that the sequences complementary to the non-translated 3' sequences of the mRNA are also efficient for inhibiting mRNA translation. Therefore, complementary oligonucleotides could be used at the non-translated 5' or 3' regions, non-coding regions of a gene in an antisense approximation to inhibit the translation of that mRNA. The oligonucleotides complementary to the non-translated 5' region of the mRNA must include the complement of the start codon AUG. The oligonucleotides complementary to the coding region of the mRNA are less efficient translation inhibitors but they could also be used according to the invention. If they are designed to hybridize with the 5' region, 3' region or the coding region of the mRNA, the antisense nucleic acids must have at least six nucleotides long and preferably have less than approximately 100 and more preferably less than approximately 50, 25, 17 or 10 nucleotides long.

Preferably, *in vitro* studies are performed first to quantify the capacity of the antisense oligonucleotides for inhibiting gene expression. Preferably these studies use controls which distinguish between antisense gene inhibition and non-specific biological effects of the oligonucleotides. Also preferably these studies compare the levels of target RNA or protein with that of an internal control of RNA or protein. The results obtained using the antisense oligonucleotides can be compared with those obtained using a control oligonucleotide. Preferably the control oligonucleotide is approximately of the same length as the oligonucleotide to be assayed and the oligonucleotide sequence does not differ from the antisense sequence more than it is deemed necessary to prevent the specific hybridization to the target sequence.

The antisense oligonucleotide can be a single or double stranded DNA or RNA or chimeric mixtures or derivatives or modified versions thereof. The oligonucleotide can be modified in the base group, the sugar group or the phosphate backbone, for example, to improve the stability of the molecule, its hybridization capacity, etc. The oligonucleotide may include other bound groups, such as peptides (for example, for directing them to the receptors of the host cells), agents for facilitating transport through the cell membrane or the blood-brain barrier, and intercalating agents. For this purpose, the oligonucleotide can be conjugated to another molecule, for example, a peptide, a transporting agent, hybridization triggered cleaving agent, etc.

The antisense oligonucleotides may comprise at least one group of modified base. The antisense oligonucleotide may also comprise at least a modified sugar group selected from the group including but not limited to arabinose, 2-fluoroarabinose, xylulose, and hexose. The antisense oligonucleotide may also contain a backbone similar to a neutral peptide. Such molecules are known as peptide nucleic acid (PNA) oligomers. In yet another embodiment, the antisense oligonucleotide comprises at least one modified phosphate backbone. In yet another embodiment, the antisense oligonucleotide is an alpha-anomeric oligonucleotide.

While antisense oligonucleotides complementary to the coding region of the target mRNA sequence can be used, those complementary to the transcribed non translated region can also be used.

In some cases, it may be difficult to reach the sufficient intracellular concentrations of the antisense to suppress the endogenous mRNA translation. Therefore, a preferred approximation uses a recombinant DNA construct in which the antisense oligonucleotide is placed under the control of a strong pol III or pol II promoter.

Alternatively, the target gene expression can be reduced by directing deoxyribonucleotide sequences complementary to the gene regulating region (i.e., the promoter and/or enhancers) to form triple helix structures preventing gene transcription in the target cells in the body. In certain embodiments, the antisense oligonucleotides are antisense morpholines.

### siRNA

Small interference RNA or siRNA are agents which are capable of inhibiting the expression of a target gene by means of RNA interference. A siRNA can be chemically synthesized, can be obtained by means of *in vitro* transcription or can be synthesized *in vivo* in the target cell. Typically, the siRNA consist of a double stranded RNA between 15 and 40 nucleotide long and may contain a 3' and/or 5' protruding region of 1 to 6 nucleotides. The length of the protruding region is independent of the total length of the siRNA molecule. The siRNA acts by means of degrading or silencing the target messenger after transcription.

The siRNA of the invention are substantially homologous to the mRNA of the LRRK2 encoding gene or to the gene sequence which encodes said protein. "Substantially homologous" is understood as having a sequence which is sufficiently complementary or similar to the target mRNA such that the siRNA is capable of degrading the latter through RNA interference. The siRNA suitable for causing said interference include siRNA formed by RNA, as well as siRNA containing different chemical modifications such as: (i) siRNA in which the bonds between the nucleotides are different than those appear in nature, such as phosphorothionate bonds; (ii) Conjugates of the RNA strand with a functional reagent, such as a fluorophore; (iii) Modifications of the ends of the RNA strands, particularly of the 3' end by means of the modification with different hydroxyl functional groups in 2' position; (iv) Nucleotides with modified sugars such as 0-alkylated residues on 2' position like 2'-O-methylribose or 2'-O-fluororibose; or (v) Nucleotides with modified bases such as halogenated bases (for example 5-bromouracil and 5-iodouracil), alkylated bases (for example 7-methylguanosine).

The siRNA can be used in the form of a double stranded RNA with the aforementioned characteristics. Alternatively, the use of vectors containing the sense and antisense strand sequence of the siRNA is possible under the control of suitable promoters for the expression thereof in the cell of interest.

Vectors suitable for expressing siRNA are those in which the two DNA regions encoding the two strands of siRNA are arranged in tandem in one and the same DNA strand separated by a spacer region which, upon transcription, forms a loop and wherein a single promoter directs the transcription of the DNA molecule giving rise to shRNA.

The siRNA can be generated intracellularly from the so called shRNA (short hairpin RNA) characterized in that the antiparallel strands forming the siRNA are connected by a loop or hairpin region. The shRNAs can be encoded by plasmids or viruses, particularly retroviruses, and are under the control of a promoter. Promoters suitable for expressing shRNA are those indicated in the paragraph above for expressing siRNA.

The siRNA and shRNA of the invention can be obtained using a series of techniques known by the person skilled in the art. The region of the nucleotide sequence taken as a basis for designing the siRNA is not limiting and it may contain a region of the coding sequence (between the start codon and the end codon) or it may alternatively contain sequences of the non-translated 5' or 3' region preferably between 25 and 50 nucleotides long and in any position in 3' direction position with respect to the start codon. One way of designing an siRNA involves the identification of the AA(N19)TT motifs wherein N can be any nucleotide in the LRRK2 gene sequence, and the selection of those having a high G/C content. If said motif is not found, it is possible to identify the NA(N21) motif wherein N can be any nucleotide.

### DNA Enzymes

On the other hand, the invention also contemplates the use of DNA enzymes to inhibit the expression of the LRRK2 gene of the invention. DNA enzymes incorporate some of the mechanistic features of both antisense and ribozyme technologies. DNA enzymes are designed such that they recognize a particular target nucleic acid sequence similar to the antisense oligonucleotide, nevertheless like the ribozyme they are catalytic and specifically cleave the target nucleic acid.

### Ribozymes

Ribozyme molecules designed for catalytically cleaving transcription products of a target mRNA to prevent the translation of the mRNA which encodes LRRK2, the activity of which is to be inhibited, can also be used. Ribozymes are enzymatic RNA molecules capable of catalyzing specific RNA cleaving. The mechanism of ribozyme action involves a specific hybridization of a ribozyme molecule sequence to a complementary target RNA followed by an endonucleolytic cleavage event. The composition of the ribozyme molecules preferably includes one or more sequences complementary to the target mRNA and the well-known sequence responsible for cleaving the mRNA or a functionally equivalent sequence. Examples of ribozymes to be used in the present invention include hammer-head ribozymes and endoribonuclease RNA (hereinafter "Cech type ribozymes").

The ribozymes can be formed by modified oligonucleotides (for example to improve the stability, targeting, etc.) and they should be distributed to cells expressing the target gene *in vivo.* A preferred distribution method involves using a DNA construct which "encodes" the ribozyme under the control of a strong constitutive pol III or pol II promoter such that the transfected cells will produce sufficient amounts of the ribozyme to destroy the endogenous target messengers and to inhibit translation. Since the ribozymes are catalytic, unlike other antisense molecules, a low intracellular concentration is required for its efficiency.

### Inhibitory antibodies

In the context of the present invention, "inhibitory antibody" is understood as any antibody capable of binding specifically to the LRRK2 protein and inhibiting one or more of the functions of said protein. The antibodies can be prepared using any of the methods which are known by the person skilled in the art, some of which have been mentioned above. Thus, the polyclonal antibodies are prepared by means of immunizing an animal with the protein to be inhibited. The monoclonal antibodies are prepared using the method described by Kohler and Milstein et al. 1975 (Nature 256: 495-497). In the context of the present invention, suitable antibodies include intact antibodies comprising a variable antigen binding region and a constant region, "Fab", "F(ab')2" and "Fab"', Fv, scFv fragments, diabodies and bispecific antibodies. Once antibodies with LRRK2 protein binding capacity are identified, those capable of inhibiting the activity of this protein will be selected using an inhibitory agent identification assay. LRRK2 Inhibitory antibodies are commercially available, such as the anti-LRRK2 (C-terminal) antibodies produced in rabbit (L0169 and L9918 from Sigma-Aldrich), the anti-LRRK2 antibodies produced in rabbit (HPA014293 from Sigma-Aldrich), the anti-LRRK2 antibodies produced in mouse (L3044 from Sigma-Aldrich), etc.

### Inhibitory peptides

As used herein, the term "inhibitory peptide" refers to those peptides capable of binding to the LRRK2 protein and inhibiting its activity as has been explained above.

### Inhibitory chemical compounds.

Chemical compounds which work as a LRRK2 inhibitor are widely known in the state of the art. Examples of LRRK2 inhibitors include, without limiting to, the compounds disclosed in the following patent applications: US2017002000, US2016200722, WO2016036586, WO2017218843, US2018305356, WO2019012093, US2017008907 and US2017002000; the publications Hatcher, J.M. et al., 2017. Adv. Neurobiol. 14: 241-264, Reith, A. D. et al. 2014. Bioorg Med Chem Lett, 22(17): 5625-5629, Kramer, et al. 2012 ACS Chem Neurosci 3(3): 151-160 and Taymans, J.M. and Greggio, E. 2016, Curr Neuropharmacol, 14(3): 214-225. Other examples of chemical compounds which work as a LRRK2 inhibitor are aminopyrazoles, such as the Genentech compounds GNE-7915 and GNE-0877.

In a particular embodiment, the chemical compound which can be used as a LRRK2 inhibitor is 3-(4-Pyrimidinyl) Indazole (MLi-2), a dihydrobenzothiophene, a 2-(benzyloxy)-5-(2-fluoropyridin-4-yl)-N-(pyridin-3-yl)benzamide (GSK2578215A) and derivatives thereof. The MLi-2 inhibitor is available in Merck, and the GSK2578215A inhibitor is available in Glaxo Smith Kline.

The present invention relates to a LRRK2 inhibitor for use in the treatment and/or prevention of Parkinson's disease (PD), or diseases relating to PD, in a subject comprising centrosomal deficit.

As used herein, the term "treating" (or "treat" or "treatment") refers to processes involving a slowing, interrupting, arresting, controlling, stopping, reducing, or reversing the progression or severity of an existing symptom, disorder, condition, or disease, but does not necessarily involve a total elimination of all disease-related symptoms, conditions, or disorders. The treatment of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may, e.g., lead to a halt in the progression of the disorder or disease or a delay in the progression of the disorder or disease. Such a partial or complete response may be followed by a relapse. It is to be understood that a subject/patient may experience a broad range of responses to a treatment (such as the exemplary responses as described herein above). In the present invention, the condition or disorder to be treated is PD, or diseases relating to PD. Specifically, the PD is sporadic Parkinson's disease (sPD) or familial PD (fPD). In another particular embodiment, the PD is at early stages of PD.

As used herein, the term "preventing" (or "prevent" or "prevention") refers to the capacity of the LRRK2 inhibitor to minimize or hinder the progression of a disease, condition or disorder in a subject. In the present invention, the condition or disorder to be prevented is PD, or diseases relating to PD. Specifically, the PD is sporadic Parkinson's disease (sPD) or familial PD (fPD). In another particular embodiment, the PD is at early stages of PD.

As used herein, the term "Parkinson's disease" (PD) refers to a neurodegenerative movement disorder diagnosed according to the effective/updated neurological diagnostic criteria or the International Parkinson and Movement Disorder Society (MDS) criteria or the two main previous sets of diagnostic criteria [United Kingdom PD Society Brain Bank (Gibb WR and Lees A.J. 1988. J Neurol Neurosurg Psychiatry, 51:745-52) and Gelb's criteria (Gelb et al., 1999. Arch Neurol., 56:33-9)] that are still used by neurologists. The disease is often characterized by motor parkinsonism symptoms with bradykinesia (slowed movement), plus resting tremors and/or stiffness (rigidity). In an advance stages, the PD is further characterized by the loss of dopaminergic neurons in the substantia nigra of the basal ganglia and the presence of Lewy bodies (LB) in postmortem brain tissues. Often, cognitive dysfunction or dementia are associated to this disease. The most common form of PD is known as sporadic Parkinson's disease (sPD), also known as idiopathic Parkinson's disease (IPD). In a particular embodiment, the PD is sporadic PD (sPD) or familial PD (fPD). In another particular embodiment, the PD is an early stage of PD.

Early PD could be divided into three stages: preclinical PD in which the neurodegenerative process is started, without evident symptoms or signs of the disease; prodromal PD in which symptoms and signs of this disease are present, but insufficient to define a full clinical picture of PD; and clinical PD in which the diagnosis of PD is achieved, based on the presence of classical motor signs (according to MDS-PD criteria, or the brain bank Criteria or Gelb criteria).

Prodromal disease refers to the stage wherein early symptoms or signs of PD neurodegeneration are present, but a clinical diagnosis is not yet possible. These criteria, which have been developed for research purposes only, include a combination of indicators ranging from mild motor symptoms [i.e., UPDRS-1987 version score ≥ 3 excluding action tremor; or MDS-UPDRS score > 6 excluding postural-action tremor], non-motor symptoms (i.e., REM SBD, olfactory dysfunction, constipation, excessive daytime somnolence, symptomatic hypotension, erectile/urinary dysfunction, depression), and ancillary diagnostic tests (i.e., abnormal tracer uptake of the presynaptic dopaminergic system: SPECT or positron emission tomography). As the skilled person in the art understands, these criteria represent only a first step in the correct description of early stages of PD, and will require constant updating as more information becomes available.

The present invention also relates to a LRRK2 inhibitor for use in the treatment and/or prevention of diseases relating to Parkinson's disease PD in a subject comprising centrosomal deficit.

The term "diseases related to PD" refers to those diseases which, as a consequence of progressive degeneration of neurons, mimic the symptoms of PD such as autonomic dysfunction, tremors, slow movement, muscle rigidity etc. Examples of diseases related to PD include, without limiting to, Multiple System Atrophy (MSA), progressive supranuclear palsy and corticobasal degeneration, dementia with Lewy bodies, or frontotemporal dementia (FTD) (Espay, A. & Litvan, I. 2011. J. Mol. Neurosci. 45(3): 343-349).

The subject to be treated with the LRRK2 inhibitor can be any subject as long as the subject comprises centrosomal deficit.

As used herein, the term "subject" refers to human and non-human animals, such as veterinary subjects. The term "non-human animal" includes all vertebrates, e.g., mammals and non-mammals, such as non-human primates, mice, rabbits, sheep, dog, cat, horse, cow, chickens, amphibians, and reptiles. In a particular embodiment, the subject is a human, man or woman of any age or race.

The subject object of the present invention is characterized by comprising centrosomal deficit. As used herein, the term "centrosomal deficit" refers to any centrosomal alteration including, without limiting to, deficits in ciliogenesis, deficits in centrosomal maturation, deficits in centrosome positioning and/or deficits in centrosome cohesion.

Deficits in ciliogenesis may be measured by quantifying the percentage of cells displaying cilia and/or alterations in cilia length (stained with antibodies against ciliary markers, such as polyglutamylated tubulin, Arl13B and others).

Deficits in centrosomal maturation is determined by measuring the integrated fluorescence intensity of centrosomes in cells displaying one centrosome, and stained with markers including pericentrin and gamma tubulin.

Deficits in centrosomal positioning in cells displaying one centrosome are measured in polarized cell types, where the centrosome is not localized properly with respect to for example the outgrowing neurite, with concomitant deficits in neurite outgrowth.

In a particular embodiment, the centrosomal deficit is a centrosomal cohesion deficit. As used herein the term "centrosomal cohesion deficit" refers to a difference in the average distance between two duplicated centrosomes, which can be expressed as differences in the mean distance of duplicated centrosomes, or as the percentage of centrosome splitting, where the percentage of cells displaying duplicated centrosomes with a distance bigger than the mean distance in control conditions is normalized to the total amount of cells displaying duplicated centrosomes. In both cases, the readout is quantified over at least 100 cells.

An assay to determine if a given subject suffers a centrosomal cohesion deficit comprises using a refined staining protocol involving antibodies against two centrosomal markers. Examples of centrosomal markers include, without being limited to, gamma tubulin, centrin, pericentrin and ninein. Antibodies against these markers are commercially available or can be easily produced by techniques widely known by the skilled person in the art.

Additionally, in a particular embodiment, the subject object of the present invention is also characterized by not comprising (not carrying) in his/her genome the replacement of a Glycine (G) with a Serine (S) in the LRRK2 protein at homologous position to position 2019 of sequence SEQ ID NO: 1, hereinafter "G2019S LRRK2 mutation". The sequence SEQ ID NO: 1 has been disclosed above.

As used throughout the present document, the expression "G2019S LRRK2 mutation" refers to the amino acid substitution of a Glycine (G) by a Serine (S) in the LRRK2 protein at the homologous position to position 2019 of sequence SEQ ID NO: 1. In the context of the present invention, the term "mutation" refers to a change in the amino acid sequence of a protein.

As the skilled person in the art knows, homologous positions between sequences can be determined by the optimal alignment of both sequences, using software widely known in the state of the art, as those disclosed above for the "sequence identity".

In another particular embodiment, the subject comprises the replacement of a Glycine (G) with a Serine (S) in the LRRK2 protein at the homologous position to position 2019 of sequence SEQ ID NO: 1 when the PD is an early stage of PD. The terms "PD" and "early stage of PD" have been defined in previous paragraphs.

In another particular embodiment, the subject comprises at least one mutation in the LRRK2 protein different from the G2019S mutation as defined above.

In another particular embodiment, the mutation in the LRRK2 protein different from the G2019S mutation is selected from the list consisting of
- the replacement of an Arginine (R) with a Cysteine (C) at homologous position to position 1441 of sequence SEQ ID NO: 1, and/or
- the replacement of an Arginine (R) with a Glycine (G) at homologous position to position 1441 of sequence SEQ ID NO: 1, and/or
- the replacement of an Arginine (R) with a Histidine (H) at homologous position to position 1441 of sequence SEQ ID NO: 1, and/or
- the replacement of an Asparagine (N) with a Histidine (H) at homologous position to position 1437 of sequence SEQ ID NO: 1, and/or
- the replacement of an Arginine (R) with a Proline (P) at homologous position to position 1628 of sequence SEQ ID NO: 1 [R1628P mutation],
- the replacement of an Tyrosine (Y) with a Cysteine (C) at homologous position to position 1699 of sequence SEQ ID NO: 1 [Y1699C mutation],
- the replacement of a Methionine (M) with a Threonine (T) at homologous position to position 1646 of sequence SEQ ID NO: 1 [M1646T mutation], and/or
- the replacement of an Isoleucine (I) with a Threonine (T) at homologous position to position 2020 of sequence SEQ ID NO: 1, and/or
- the replacement of an Adenine (A) with a Valine (V) at homologous position to position 419 of sequence SEQ ID NO: 1 [A419V mutation], and/or
- the replacement of a Glycine (G) with an Arginine (R) at homologous position to position 2385 of sequence SEQ ID NO: 1 [G2385R mutation], and/or
- the replacement of a Tyrosine (Y) with a Cysteine (C) at homologous position to position 2189 of sequence SEQ ID NO: 1 [Y2189C mutation],
- the replacement of an Arginine (R) with a Histidine (H) at homologous position to position 1398 of sequence SEQ ID NO: 1,
- the replacement of an Asparagine (N) with a Lysine (K) at homologous position to position 551 of sequence SEQ ID NO: 1 [N551K mutation],
- the replacement of an Arginine (R) with a Histidine (H) at homologous position to position 1398 of sequence SEQ ID NO: 1 [R1398H mutation],
- the replacement of an Glycine (G) with an Arginine (R) at homologous position to position 2385 of sequence SEQ ID NO: 1 [G2385R mutation],
- the replacement of an Isoleucine (I) with a Valine (V) at homologous position to position 1371 of sequence SEQ ID NO: 1 [I1371V mutation],
- the replacement of a Methionine (M) with a Threonine (T) at homologous position to position 2397 of sequence SEQ ID NO: 1 [M2397T mutation],
- the replacement of an Isoleucine (I) with a Valine (V) at homologous position to position 2081 of sequence SEQ ID NO: 1 [N2081D mutation],
- the replacement of an Asparagine (N) with an Aspartic acid (D) at homologous position to position 1371 of sequence SEQ ID NO: 1 [N1371D mutation],
- the replacement of an Arginine (R) with a Threonine (T) at homologous position to position 1413 of sequence SEQ ID NO: 1 [R1413T mutation].
- the replacement of an Arginine (R) with a Lysine (K) at homologous position to position 1707 of sequence SEQ ID NO: 1 [R1707K mutation] and/or
- combinations thereof.

Additional particular embodiments of mutations in the LRRK2 protein different from the G2019S mutation can be found below in table 1.

**Table 1 - Mutations in LRRK2. Nuytemans K, Theuns, J., Van Broeckhoven C. Genetic etiology of Parkinson's disease associated with mutations in the SNCA, PARK2, PINK1, PARK7 and LRRK2 genes: an update. Human Mutation 31: 763-780, 2010 (PubMed ID: 20506312). Cruts M, Theuns J, Van Broeckhoven C. Locus-specific mutation databases for neurodegenerative brain diseases. Human Mutation 33: 1340-1344, 2012 doi: 10.1002/humu.22117 (PubMed ID: 22581678).**

| **Mutation** | **Exon/Domain** |
|---|---|
| *LRRK2* c.-44G>T (g.5078G>T) | EX1 |
| *LRRK2* c.-30G>C (g.5092G>C) | EX1 |
| *LRRK2* Glu10Lys (g.5149G>A) | EX1/N-term |
| *LRRK2* Ser52Phe (g.5548C>T) | EX2/N-term |
| *LRRK2* Leu119Pro (g.15624T>C) | EX4/N-term |
| *LRRK2* Leu153 (g.17979T>C) | EX5/N-term |
| *LRRK2* Lys182 (g.18068A>G) | EX5/N-term |
| *LRRK2* Ala211Val (g.20533C>T) | EX6/N-term |
| *LRRK2* Cys228Ser (g.20584G>C) | EX6/N-term |
| *LRRK2* His275 (g.23658T>C) | EX7/N-term |
| *LRRK2* Asn289 (g.29844C>T) | EX8/N-term |
| *LRRK2* Glu334Lys (g.31263G>A) | EX9/N-term |
| *LRRK2* Asn363Ser (g.31351A>G) | EX9/N-term |
| *LRRK2* Val366Met (g.31359G>A) | EX9/N-term |
| *LRRK2* IVS9-10C>A (g.31445G>A) | IVS9 |
| *LRRK2* IVS10-4A>G (g.32896A>G) | IVS10 |
| *LRRK2* Ser461 (g.37332T>C) | EX12/N-term |
| *LRRK2* Leu488 (g.39515A>T) | EX13/N-term |
| *LRRK2* Arg506Gln (g.39568G>A) | EX13/N-term |
| *LRRK2* Lys544Glu (g.43865A>G) | EX14/N-term |
| *LRRK2* Lys616Arg (g.054889A>G) | EX16/N-term |
| *LRRK2* IVS16-14delT (g.57864deIT) | IVS16 |
| *LRRK2* Val674 (g.57958A>C) | EX17/N-term |
| *LRRK2* Tyr707 (g.58131C>T) | EX18/Ankyrin-like |
| *LRRK2* Met712Val (g.58144A>G) | EX18/Ankyrin-like |
| *LRRK2* Ala716Val (g.58157C>T) | EX18/Ankyrin-like |
| *LRRK2* Ile723Val (g.58177A>G) | EX18/Ankyrin-like |
| *LRRK2* Pro755Leu (g.63887C>T) | EX19/Ankyrin-like |
| *LRRK2* Arg793Met (g.64001G>T) | EX19/Ankyrin-like |
| *LRRK2* IIe810Val (g.64051A>G) | EX19/Ankyrin-like |
| *LRRK2* Ser827 (g.64106T>C) | EX19/Ankyrin-like |
| LRRK2 IVS19+5_8delGTAA (g.64128_64131delGTAA) | IVS19 |
| LRRK2 IVS19-9_8insT (g.67332_67333insT) | IVS19 |
| LRRK2 Lys871Glu (g.67451A>G) | EX20/L1 |
| *LRRK2* Gln923His (g.73614G>C) | EX21/L1 |
| LRRK2 Leu929 (g.073632G>A) | EX21/L1 |
| LRRK2 Gln930Arg (g.73634A>G) | EX21/L1 |
| LRRK2 Leu953 (g.74883T>C) | EX22/L1 |
| LRRK2 Ser973Asn (g.75456G>A) | EX25/L1 |
| LRRK2 IIe1006Met (g.75556A>G) | EX23/LRR |
| LRRK2 Arg1067Gln (g.78336G>A) | EX24/LRR |
| LRRK2 Ser1096Cys (g.78423C>G) | EX24/LRR |
| LRRK2 Gln1111His (g.78469G>T) | EX24/LRR |
| LRRK2 Leu1114 (g.78478A>G) | EX24/LRR |
| LRRK2 IIe1122Val (g.79115A>G) | EX25/LRR |
| LRRK2 Ala1151Thr (g.79202G>A) | EX25/LRR |
| LRRK2 Leu 1165Pro (g.79245T>C) | EX25/LRR |
| LRRK2 IVS25-8delT (g.82771deIT) | IVS25 |
| LRRK2 IIe1192Val (g.82856A>G) | EX26/LRR |
| LRRK2 His1216Arg (g.83994A>G) | EX27/LRR |
| LRRK2 Ser1228Thr (g.84030G>C) | EX27/LRR |
| LRRK2 IVS27-9C>T (g.85764C>T) | IVS27 |
| LRRK2 Pro1262Ala (g.85781C>G) | EX28/LRR |
| LRRK2 Arg1325Gln (g.88471G>A) | EX29/L2 |
| LRRK2 Arg1398 (g.089100T>G) | EX30/ROC |
| LRRK2 Thr1410Met (g.89135C>T) | EX30/ROC |
| LRRK2 Asp1420Asn (g.89164G>A) | EX30/ROC |
| LRRK2 Lys1423 (g.89175G>A) | EX30/ROC |
| LRRK2 Ala1430 (g.89196C>T) | EX30/ROC |
| LRRK2 IVS30+12delT (g.89235deIT) | IVS30 |
| LRRK2 IVS30-6C>T (g.90415C>T) | IVS30 |
| LRRK2 Arg1441 (g.90426C>T) | EX31/ROC |
| LRRK2 Ala1442Pro (g.90427G>C) | EX31/ROC |
| LRRK2 Pro1446Leu (g.90440C>T) | EX31/ROC |
| LRRK2 Val1450IIe (g.90451G>A) | EX31/ROC |
| LRRK2 Lys1468Glu (g.90505A>G) | EX31/ROC |
| LRRK2 Arg1483Gln (g.90551G>A) | EX31/ROC |
| LRRK2 IVS31+3A>G (g.90642A>G) | IVS31/COR |
| LRRK2 Arg1514Gln (g.93966G>A) | EX32/COR |
| LRRK2 Pro1542Ser (g.94049C>T) | EX32/COR |
| LRRK2 IVS32+14G>A (g.94177G>A) | IVS32 |
| LRRK2 IVS33+6T>A (g.95296T>A) | IVS33 |
| LRRK2 Val1613Ala (g.99988T>C) | EX34/COR |
| LRRK2 Gly1624 (g.100022C>A) | EX34/COR |
| LRRK2 Ser1647Thr (g.100089T>A) | EX34/COR |
| LRRK2 Arg1725Gln (g.102028G>A) | EX36/COR |
| LRRK2 Arg1728His (g.102037G>A) | EX36/COR |
| LRRK2 Arg1728Leu (g.102037G>T) | EX36/COR |
| LRRK2 Leu 1795Phe (g.102376G>T) | EX37/COR |
| LRRK2 Gly1819 (g.102448T>C) | EX37/COR |
| LRRK2 Gln1823Lys (g.102458C>A) | EX37/COR |
| LRRK2 IVS37-9A>G (g. 103141A>G) | IVS37 |
| *LRRK2* Met1869Val (g.103245A>G) | EX38/COR |
| *LRRK2* Met1869Thr (g.103246T>C) | EX38/COR |
| *LRRK2* Leu1870Phe (g.103250G>T) | EX38/COR |
| *LRRK2* Glu1874Stop (g.103260G>T) | EX38/COR |
| *LRRK2* IVS38+7C>T (g.103303C>T) | IVS38 |
| *LRRK2* Arg1941His (g.115021G>A) | EX40/Kinase |
| *LRRK2* Leu2005 (g.120348C>T) | EX41/Kinase |
| *LRRK2* Tyr2006His (g.120351T>C) | EX41/Kinase |
| *LRRK2* IIe2012Thr (g.120370T>C) | EX41/Kinase |
| *LRRK2* Tyr2018 (g.120389C>T) | EX41/Kinase |
| *LRRK2* Gly2019Ser (g.120390G>A) | EX41/Kinase |
| *LRRK2* Thr2031Ser (g.120426A>T) | EX41/Kinase |
| *LRRK2* Glu2108 (g.128442G>A) | EX43/Kinase |
| *LRRK2* Cys2139Ser (g.131562T>A) | EX44/L3 |
| *LRRK2* Thr2141Met (g.131569C>T) | EX44/L3 |
| *LRRK2* Arg2143His (g.131575G>A) | EX44/WD40 |
| *LRRK2* Gly2170 (g.131657C>A) | EX44/WD40 |
| *LRRK2* Asp2175His (g.131670G>C) | EX44/WD40 |
| *LRRK2* IVS46-14T>A (g.139236T>A) | IVS46 |
| *LRRK2* IVS46-8delT (g.139242delT) | IVS46 |
| *LRRK2* IVS47-9delT (g.143383delT) | IVS47 |
| *LRRK2* Thr2356IIe (g.143430C>T) | EX48/WD40 |
| *LRRK2* Gly2385 (g.143518A>G) | EX48/WD40 |
| *LRRK2* Val2390Met (g.143531G>A) | EX48/WD40 |
| *LRRK2* Glu2395Lys (g.144833G>A) | EX49/WD40 |
| *LRRK2* Gly2432 (g.144946C>G) | EX49/WD40 |
| *LRRK2* Leu2439IIe (g.144965C>A) | EX49/WD40 |
| *LRRK2* Leu2466His (g.147002T>A) | EX50/WD40 |
| *LRRK2* Glu2490fs (g.147639delC) | EX51/WD40 |
| LRRK2 Ala1440Pro (G>C) | - |

In another particular embodiment, the subject comprises increased expression levels of the gene encoding the LRRK2 protein in PD patients with centrosome deficit with respect to a reference level in control individuals. Methods for determining the expression levels of a gene are widely known in the state of the art, and any of them can be used in the context of the present invention. The determination of the expression levels of gene encoding the LRRK2 protein may comprise measuring the level of cDNA, the level of mRNA, and/or the level of the protein encoded by said gene. By way of illustration and not limitation, the levels of mRNA of said gene can be quantified by means of using conventional methods, for example, methods comprising the amplification of mRNA and the quantification of the product of the amplification of said mRNA, such as electrophoresis and staining, or alternatively, by means of Southern blot and the use of suitable probes, Northern blot and the use of specific probes of mRNA of the gene of interest (gene encoding the LRRK2 protein) or of the corresponding cDNA thereof, S1 nuclease mapping, hybridisation, RT-Q-PCR, the micro-arrays and RNAsequencing, etc. If the quantification of the expression of gene encoding the LRRK2 protein is going to be carried out from the protein, i.e. the LRRK2 protein, then the isolated biological sample of the subject must be treated to extract the proteins. Methods for extracting or isolating proteins are known by the person skilled in the art and they are commercially available. The levels of gene encoding the LRRK2 protein can be quantified by means of any conventional method that enables said protein to be detected and quantified in a sample of a subject. By way of illustration and not limitation, the levels of said protein can be quantified, for example, by means of the use of antibodies with the capacity to bind specifically to the LRRK2 protein and the subsequent quantification of the complexes formed. Examples of antibodies with the capacity to specifically bind to the LRRK2 protein have been disclosed in previous paragraphs.

The term "reference level" refers to the expression levels of the gene encoding the LRRK2 protein in a sample of a subject not suffering from PD and not comprising centrosomal deficit.

Additionally, in another particular embodiment, the subject, further to comprise centrosomal deficit, comprises nucleotide changes [such as SNPs] in other genes different from the gene encoding the LRRK2 protein, or in the upstream and downstream 5 kilobases-length sequences flanking the gene encoding the LRRK2 protein (Mata, I. et al. 2012. Mov Disord. 27(14): 1823-1826), which are also indicative of PD. Examples of these nucleotide changes include, without limiting to, the SNPs disclosed in table 2 (next page) and in the database PDGene meta-analysis for rs76904798 (LRRK2, chr12:40590546-40763087 +/-50000 base pairs): T vs. C. (The Michael J. Fox Foundation for Parkinson's disease).

**Table 2: Loci associated with Parkinson's disease (Nalls, M. et al. 2018. bioRxiv 388165; doi: https://doi.org/10.1101/388165).**

| **SNP** | **CHR** | **BP** | **Nearest Gene** | **Effect allele** | **Other allele** | **Effect allele frequency** |
|---|---|---|---|---|---|---|
| rs6658353 | 1 | 161469054 | FCGR2A | c | g | 0.501 |
| rs11578699 | 1 | 171719769 | VAMP4 | t | c | 0.195 |
| rs76116224 | 2 | 18147848 | KCNS3 | a | t | 0.904 |
| rs2042477 | 2 | 96000943 | KCNIP3 | a | t | 0.242 |
| rs6808178 | 3 | 28705690 | LINC00693 | t | c | 0.379 |
| rs55961674 | 3 | 122196892 | KPNA1 | t | c | 0.172 |
| rs11707416 | 3 | 151108965 | MED12L | a | t | 0.367 |
| rs1450522 | 3 | 161077630 | SPTSSB | a | g | 0.674 |
| rs34025766 | 4 | 17968811 | LCORL | a | t | 0.159 |
| rs62333164 | 4 | 170583157 | CLCN3 | a | g | 0.326 |
| rs26431 | 5 | 102365794 | PAM | c | g | 0.703 |
| rs11950533 | 5 | 134199105 | C5orf24 | a | c | 0.102 |
| rs9261484 | 6 | 30108683 | TRIM40 | t | c | 0.245 |
| rs12528068 | 6 | 72487762 | RIMS1 | t | c | 0.284 |
| rs997368 | 6 | 112243291 | FYN | a | g | 0.805 |
| rs75859381 | 6 | 133210361 | RPS12 | t | c | 0.967 |
| rs76949143 | 7 | 66009851 | GS1-124K5.11 | a | t | 0.051 |
| rs2086641 | 8 | 130901909 | FAM49B | t | c | 0.723 |
| rs6476434 | 9 | 34046391 | UBAP2 | t | c | 0.734 |
| rs10748818 | 10 | 104015279 | GBF1 | a | g | 0.851 |
| rs7938782 | 11 | 10558777 | RNF141 | a | g | 0.878 |
| rs7134559 | 12 | 46419086 | SCAF11 | t | c | 0.404 |
| rs11610045 | 12 | 133063768 | FBRSL1 | a | g | 0.490 |
| rs9568188 | 13 | 49927732 | CAB39L | t | c | 0.740 |
| rs4771268 | 13 | 97865021 | MBNL2 | t | c | 0.230 |
| rs12147950 | 14 | 37989270 | MIPOL1 | t | c | 0.438 |
| rs3742785 | 14 | 75373034 | RPS6KL1 | a | c | 0.787 |
| rs2904880 | 16 | 28944396 | CD19 | c | g | 0.309 |
| rs6500328 | 16 | 50736656 | NOD2 | a | g | 0.599 |
| rs200564078 | 16 | 58587672 | CNOT1 | t | c | 0.003 |
| rs12600861 | 17 | 7355621 | CHRNB1 | a | c | 0.648 |
| rs2269906 | 17 | 42294337 | UBTF | a | c | 0.653 |
| rs850738 | 17 | 42434630 | FAM171A2 | a | g | 0.606 |
| rs61169879 | 17 | 59917366 | BRIP1 | t | c | 0.164 |
| rs666463 | 17 | 76425480 | DNAH17 | a | t | 0.833 |
| rs1941685 | 18 | 31304318 | ASXL3 | t | g | 0.498 |
| rs8087969 | 18 | 48683589 | MEX3C | t | g | 0.550 |
| rs77351827 | 20 | 6006041 | CRLS1 | t | c | 0.128 |
| rs2248244 | 21 | 38852361 | DYRK1A | a | g | 0.283 |

Other mutations which are also indicative of PD are comprised in the regulatory region of the LRRK2 gene, such as rs10878226, rs7294619, rs11176013, rs10878371, rs1491942 (Mata, l. *et al.* 2012. Cited *ad supra*).

The administration of the LRRK2 inhibitor to the subject comprises the administration of a therapeutically effective amount of a LRRK2 inhibitor. The term "therapeutically effective amount" means the amount of LRRK2 inhibitor that will elicit the biological or medical response of a tissue, system, animal or human that is being sought by the researcher, veterinarian, medical doctor or other clinician. In the context of the present invention, the biological or medical response to elicit is the treatment and/or prevention of PD.

The LRRK2 inhibitor of the present invention may be used in combination with one or more other drugs in the treatment, prevention, control, amelioration, or reduction of risk of PD, where the combination of the drugs together are safer or more effective than either drug alone. Thus, in particular embodiment, the inhibitor of the invention is administered in combination with another compound useful for treating PD. Such other compound(s)/drug(s) may be administered by a route and in an amount commonly used therefore, contemporaneously or sequentially with the LRRK2 inhibitor. When the LRRK2 inhibitor is used contemporaneously with one or more other drugs, a pharmaceutical composition in unit dosage form containing such other drugs and the LRRK2 inhibitor is preferred. However, the combination therapy may also include therapies in which the LRRK2 inhibitor and one or more other drugs are administered on different overlapping schedules. It is also contemplated that when used in combination with one or more other active ingredients, the LRRK2 inhibitor and the other active ingredients may be used in lower doses than when each is used singly. Accordingly, the pharmaceutical compositions of the present invention include those that contain one or more other active ingredients, in addition to a LRRK2 inhibitor.

Examples of other compounds useful for treating PD which may be used in conjunction with LRRK2 inhibitor include, without limiting to, L-DOPA; dopaminergic agonists such as quinpirole, ropinirole, pramipexole, pergolide and bromocriptine; MAO-B inhibitors such as rasagiline, deprenyl and selegiline; DOPA decarboxylase inhibitors such as carbidopa and benserazide; and COMT inhibitors such as tolcapone and entacapone; or potential therapies such as an adenosine A2a antagonists, metabotropic glutamate receptor 4 modulators, or growth factors such as brain derived neurotrophic factor (BDNF), and a pharmaceutically acceptable carrier.

The weight ratio of the LRRK2 inhibitor of the present invention to the other active ingredient(s) may be varied and will depend upon the effective dose of each ingredient. Generally, an effective dose of each will be used. Thus, for example, when the LRRK2 inhibitor of the present invention is combined with another agent, the weight ratio of the LRRK2 inhibitor to the other agent will generally range from about 1000:1 to about 1:1000, or from about 200:1 to about 1:200. Combinations of LRRK2 inhibitor and other active ingredients will generally also be within the aforementioned range, but in each case, an effective dose of each active ingredient should be used.

The LRRK2 inhibitors of the present invention may be administered by oral, parenteral (e.g., intramuscular, intraperitoneal, intravenous, ICV, intracisternal injection or infusion, subcutaneous injection, or implant), by inhalation spray, nasal, vaginal, rectal, sublingual, buccal or topical routes of administration and may be formulated, alone or together, in suitable dosage unit formulations containing conventional non-toxic pharmaceutically acceptable carriers, adjuvants and vehicles appropriate for each route of administration. In addition to the treatment of warm-blooded animals the LRRK2 inhibitors of the invention are effective for use in humans.

The LRRK2 inhibitors of the present invention can be incorporated into pharmaceutical compositions for its administration to the subject. Thus, in a particular embodiment, the inhibitor of the invention is comprised within a pharmaceutical composition. The LRRK2 inhibitors of the present invention may conveniently be presented in dosage unit form and may be prepared by any of the methods well known in the art of pharmacy. All methods include the step of bringing the active ingredient into association with the carrier which constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition the active compound is included in an amount sufficient to produce the desired effect upon the process or condition of diseases. As used herein, the term "composition" is intended to encompass a product comprising the specified ingredients in the specified amounts, as well as any product which results, directly or indirectly, from combination of the specified ingredients in the specified amounts.

The pharmaceutical compositions containing the active ingredient may be in a form suitable for oral use, for example, as tablets, troches, lozenges, aqueous or oily suspensions, dispersible powders or granules, emulsions, solutions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. Oral tablets may also be formulated for immediate release, such as fast melt tablets or wafers, rapid dissolve tablets or fast dissolve films.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxy-propylmethylcellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds of the present invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions and the like, containing the compounds of the present invention are employed. Similarly, transdermal patches may also be used for topical administration.

In the treatment, prevention, control, amelioration, or reduction of risk of sPD, an appropriate dosage level of the LRRK2 inhibitor of the invention will generally be about 0.01 to 500 mg per kg patient body weight per day which can be administered in single or multiple doses. A suitable dosage level may be about 0.01 to 250 mg/kg per day, about 0.05 to 100 mg/kg per day, or about 0.1 to 50 mg/kg per day. Within this range the dosage may be 0.05 to 0.5, 0.5 to 5 or 5 to 50 mg/kg per day. For oral administration, the compositions may be provided in the form of tablets containing 1.0 to 1000 milligrams of the active ingredient, particularly 1.0, 5.0, 10.0, 15.0. 20.0, 25.0, 50.0, 75.0, 100.0, 150.0, 200.0, 250.0, 300.0, 400.0, 500.0, 600.0, 750.0, 800.0, 900.0, and 1,000.0 milligrams of the LRRK2 inhibitor for the symptomatic adjustment of the dosage to the patient to be treated. The compounds may be administered on a regimen of 1 to 4 times per day, or may be administered once or twice per day.

It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

As explained in the beginning of the present description, the present invention allows the skilled person in the art to identify those PD patients who can benefit from LRRK2-related therapeutic interventions as LRRK2 inhibitors.

Therefore, in another aspect, the present invention relates to an *in vitro* method for designing a customized therapy for a subject with PD, or diseases related to PD, hereinafter "method of the invention", comprising determining the presence of centrosomal deficit in a biological sample isolated from said subject, and wherein if the subject shows centrosomal deficit and does not carry the G2019S mutation in the LRRK2 protein, then the subject is susceptible to receive a therapy based on a LRRK2 inhibitor.

The terms "subject", "centrosomal deficit", "Parkinson's disease", "sporadic Parkinson's disease", "familial Parkinson's disease", "diseases related to PD", and "LRRK2 protein" and "LRRK2 transcripts" have been explained above in the present description, and these explanations are applicable to the present inventive aspect.

In a particular embodiment, the centrosomal deficit is centrosomal cohesion deficit. The term "centrosomal cohesion deficit" has been explained previously in the present description for the LRRK2 inhibitor and it is application to the present inventive aspect. In order to detect centrosomal cohesion deficit, the cells in the sample have to be in proliferation phase, if not, the cells have to be stimulated to get proliferation. Methods for stimulating cells and get proliferation are widely known in the state of the art.

In another particular embodiment, the method of the invention comprises determining the presence of the replacement of a Glycine (G) with a Serine (S) in the LRRK2 protein at homologous position to position 2019 of sequence SEQ ID NO: 1 (G2019S mutation) in a biological sample isolated from said subject, wherein if the subject does not comprise the G2019S mutation in the LRRK2 protein, then the subject is susceptible to receive a therapy based on a LRRK2 inhibitor.

Methods for determining if a subject comprises centrosomal deficit, as well as the presence of the G2019S LRRK2 mutation, in a biological sample have been explained above in the present description.

In another particular embodiment, the diseases related to PD are Multiple System Atrophy, progressive supranuclear palsy and corticobasal degeneration, dementia with Lewy bodies, or frontotemporal dementia.

In another particular embodiment, the PD is sporadic PD (sPD) or familiar PD (fPD).

The present method can be applied to any type of biological sample from a subject. As use herein, the term "biological sample" refers to any material comprising a nucleic acid. Examples of biological samples include, but without limiting to, a biopsy sample, a tissue (e.g. brain tissue), cell or fluid (e.g. serum, saliva, semen, sputum, cerebral spinal fluid (CSF), tears, mucus, sweat, brain extracts and the like) and blood (e.g. PBMCs (peripheral blood-derived mononuclear cells) such as neutrophils, monocytes).

As the skilled person knows, determining the presence of centrosomal deficit involves the use of cells. In order to do this, the biological sample obtained from the subject is processed, obtaining isolated cells. Example of cells which can be used for determining the presence of centrosomal deficit include, without limiting to, cells derived from the periphery, e.g. PBMCs from blood, purified monocytes or neutrophils from blood, immortalized lymphocytes derived from blood (LCLs), etc; sperm cells; epitelial cells; fibroblast cells; and skin cells. In a particular embodiment, the biological sample is peripheral blood-derived cells, preferably, peripheral blood mononuclear cell (PBMC), purified monocytes or neutrophils. In a more particular embodiment, the cells used are immortalized lymphocytes derived from blood. As indicated above, in order to detect centrosomal deficit, the cells in the sample can be stimulated to get proliferation. Methods for stimulating cells and get proliferation are widely known in the state of the art.

The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated.

In a particular embodiment of the method of the invention, LRRK2 protein comprises an amino acid sequence having a sequence identity of at least 60, 65, 70, 75, 80, 85, 90, 95, 96, 97, 98, 99% with the SEQ ID NO: 1. In another particular embodiment, the LRRK2 protein comprises an amino acid sequence having a sequence identity of at least 100% with the SEQ ID NO: 1. The terms "sequence identity" as well as "LRRK2 protein" and the sequence "SEQ ID NO: 1" have been defined previously in the present description. In a more particular embodiment, the LRRK2 protein consists of the sequence SEQ ID NO: 1.

The method of invention relates to an *in vitro* method for designing a customized therapy for a subject with PD. In a particular embodiment, the subject is at early stage of PD. The term "early stage of PD" has been defined above in previous paragraphs.

Once it is concluded that the subject shows centrosomal deficit and/or does not carry the G2019S mutation in the LRRK2 protein and/or the expression levels of the gene encoding the LRRK2 protein are increased, then the subject is susceptible to receive a therapy based on a LRRK2 inhibitor. Examples of LRRK2 inhibitors have been disclosed with respect to the inhibitor of the invention, and they are applicable to the present method.

Therefore, in another particular embodiment of the method of the invention, the LRRK2 inhibitor is selected from the group consisting of a chemical compound, a LRRK2 specific siRNA, a LRRK2 specific antisense oligonucleotide, a LRRK2 specific ribozyme and a LRRK2 specific antibody. Examples of each of these LRRK2 inhibitors have been provided and explained in previous paragraphs of the present description. In another particular embodiment of the method of the invention, the chemical compound is 3-(4-Pyrimidinyl) Indazole (MLi-2), a dihydrobenzothiophene, a 2-(benzyloxy)-5-(2-fluoropyridin-4-yl)-N-(pyridin-3-yl)benzamide (GSK2578215A), and derivatives thereof.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### DESCRIPTION OF THE DRAWINGS

Figure 1. Centrosomal cohesion quantified from three controls and three G2019S LRRK2-PD patient lymphoblasts, and FACS analysis.
Figure 2. Revised staining protocol using two centrosomal markers and quantification of centrosomal cohesion from three control and three G2019S LRRK2-PD patient lymphoblasts.
Figure 3. Dose-response and washout of MLi2 and effects on centrosomal cohesion from five control and five G2019S LRRK2-PD patient lymphoblasts.
Figure 4. Centrosomal cohesion deficits in G2019S-LRRK2 PD patient lymphoblasts and sporadic PD lymphoblasts as compared to control, and reversal by MLi2.

### EXAMPLES

### Example 1

### I. MATERIAL AND METHODS

### Study participants

Subjects were recruited at the Movement Disorder Unit of the Lille University Hospital with written informed consent, and the study was approved by the local ethics committee. Patients were examined by neurologists specialized in movement disorders. Subjects participating in the study donated blood samples for DNA extraction, routine LRRK2 genotyping and lymphocyte immortalization. Seven patients heterozygous for the G2019S LRRK2 mutation, 13 sporadic PD patients and 13 unrelated, gender- and age-matched controls were included.

### Peripheral blood mononuclear cell (PBMC) isolation and transformation

Blood samples were collected in BD Vacutainer CPT Cell Preparation Tubes containing sodium heparin (Le Pont-de-Claix, France). Peripheral blood mononuclear cells (PBMCs) were collected and processed according to manufacturer's instructions, and lymphocytes were immortalized by infection with Epstein-Barr virus (EBV) as described. Briefly, cell lines were established from freshly isolated or cryopreserved lymphocytes using standard EBV transformation protocols which include cell separation by gradient centrifugation and lymphocyte growth enhancement by 1% (v/v) of the mitogenic phytohemagglutinin-M (PHA-M, ThermoFisher Scientific, 10576015). Cell lines were maintained in RPMI 1640 medium supplemented with 20 % fetal bovine serum, 2 % L-glutamine, 20 units/mL penicillin and 20 µg/ml streptomycin in T75 flasks in 5 % CO₂ at 37 °C. Cells were maintained at a density of 10⁶ cells/mL, with cell density monitored every other day using trypan blue staining.

### Immunofluorescence and laser confocal imaging

Coverslips (13 mm diameter) were placed into 24-well plates and coated with Cell-Tak Cell and Tissue Adhesive solution (Corning, nr. 354240) according to manufacturer's instructions. After 20 minutes incubation at room temperature, the solution was removed, and coverslips were rinsed twice with distilled water followed by air-drying. Lymphoblast cells (200,000/well) were added to dry coated coverslips, and cells attached by slight centrifugation at 690 g for 10 minutes at room temperature (without brake). Cells were fixed with 2 % paraformaldehyde (PFA) in phosphate-buffered saline (PBS) for 20 minutes at room temperature, followed by 5 min of ice-cold methanol fixation before permeabilization with 0.2% Triton-X100/PBS for 20 min. After blocking for 1 hour in 0.5% BSA (w/v) in 0.2% Triton-X100/PBS (blocking buffer), cells were incubated with primary antibodies overnight at 4 °C in blocking buffer. Primary antibodies included mouse monoclonal anti-γ-tubulin (1:1000; Abcam ab11316), rabbit polyclonal anti-pericentrin (1:1000; Abcam ab4448) or rabbit monoclonal anti-phospho-Rab10 (1:100; Abcam ab237703). The following day, coverslips were washed three times 10 minutes in 0.2% Triton-X100/PBS, followed by incubation with secondary antibodies (Alexa488-conjugated goat anti-mouse (1:1000; Invitrogen) and Alexa594-conjugated goat anti-rabbit (1:1000; Invitrogen)) in blocking buffer for 1 hour. Coverslips were washed three times in 0.2% Triton-X100/PBS, rinsed in PBS and mounted in mounting medium with DAPI (Vector Laboratories). For staining with the anti-phospho-Rab10 antibody, the methanol fixation was omitted, and 0.2% Triton-X100 was replaced by 0.1% saponin throughout. For treatment of cells with MLi2, cells were seeded on coated coverslips as described above, and incubated with or without MLi2 (generous gift of D. Alessi, University of Dundee, UK) (or DMSO as control) at the indicated concentrations for two hours at 37 °C, before attachment by slight centrifugation followed by fixation as described above.

Images were acquired on a Leica TCS-SP5 confocal microscope using a 63X 1.4 NA oil UV objective (HCX PLAPO CS). Images were collected using single excitation for each wavelength separately, and dependent on secondary antibodies (488 nm Argon Laser line and a 498-551 nm emission band pass; 543 HeNe laser line and a 615-671 nm emission band pass; 405 nm UV diode and a 418-467 nm emission band pass). Around 30 image sections of selected areas were acquired with a step size of 0.5 µm, and z-stack images analyzed and processed using Leica Applied Systems (LAS AF6000) image acquisition software. The same laser intensity settings, exposure times and zoom were used for image acquisition of individual experiments to be quantified.

The mean distance between duplicated centrosomes in control cells was 1.3 ± 0.2 µm (mean ± s.e.m., n=10 cells), and duplicated centrosomes were scored as being separated when the distance was > 1.3 µm. Only cells which displayed clear centrosomal staining by both the anti-pericentrin and anti-γ-tubulin antibodies were analyzed, and in all cases, mitotic cells were excluded from this analysis. For each sample, minimally 100 cells were quantified by an observer blind to condition. Additionally, some experimental conditions were independently quantified by an additional two observers blind to condition, with identical results obtained in all cases.

Quantification of the phospho-Rab10 signal was performed over non-processed and non-saturated images acquired during the same day with the same laser intensities. The percentage of cells diplaying presence of a phospho-Rab10 signal colocalizing with a centrosomal marker was quantified from 100-150 cells per condition and experiment.

### Statistical analysis

Data were checked for normal distribution using the Shapiro-Wilk test. One-way ANOVA with Tukey's post-hoc test was employed, with significance set at P < 0.05. Non-normally distributed data were analyzed by Kruskal-Wallis test and Dunn's multiple comparison. Spearman correlations were used to determine associations between levels of LRRK2 or LRRK2 Ser935 and Rab10 Thr73. All statistical analysis and graphs employed the use of Prism software version 7.0 (GraphPad, San Diego, CA).

### II. RESULTS

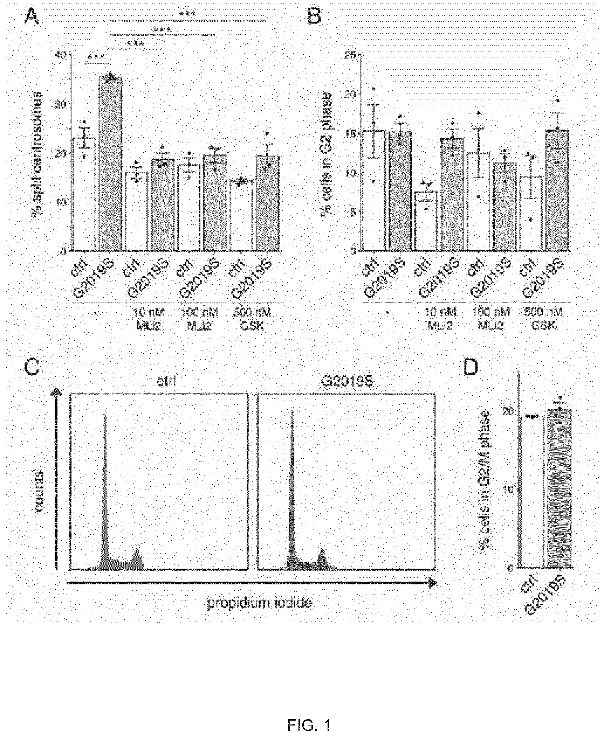

Since peripheral blood-derived lymphocytes are easily obtained, can be EBV-transformed, and have been shown to express high levels of LRRK2, we chose those cells for further analysis. Centrosomal cohesion deficits were observed in three lymphoblast lines from G2019S LRRK2 PD patients as compared to three healthy age- and sex-matched controls, could be reversed by short-term application of two distinct LRRK2 kinase inhibitors (MLi2, GSK2578215A) (Figure 1A), and were not associated with cell cycle-dependent alterations (Figures 1B to 1D).

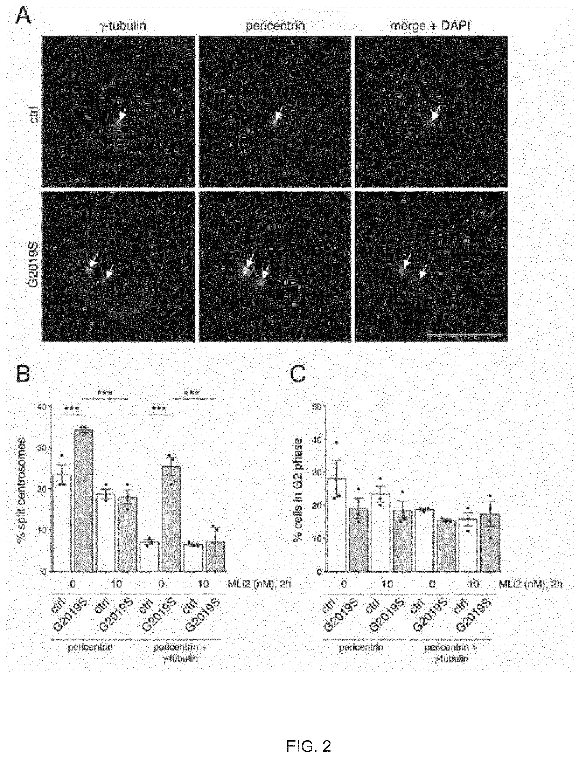

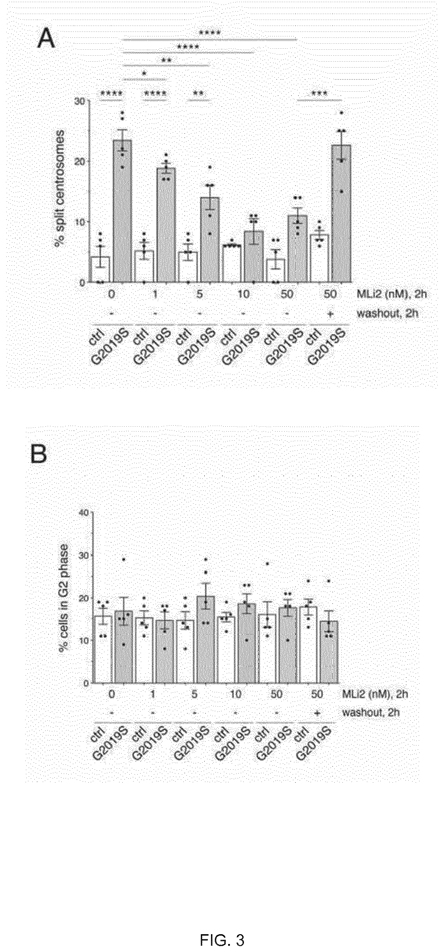

Using a refined staining protocol involving antibodies against two centrosomal markers (Figures 2A to 2C), we corroborated the centrosomal cohesion deficits in five G2019S LRRK2 PD patients as compared to five healthy controls (Figures 3A and 3B). Dose-response experiments revealed that a significant reversal of the cohesion deficit could already be observed with as low as 1 nM MLi2, with complete reversal observed at 10 nM MLi2 (Figures 3A and 3B). Upon removal of kinase inhibitor, the centrosomal cohesion deficits in G2019S LRRK2 PD samples reverted back to the ones observed in the absence of inhibitor, indicating the highly dynamic nature of the phenotype in a manner dependent on LRRK2 kinase activity (Figures 3A and 3B).

To obtain larger sample sets, additional lymphoblast lines were generated by the laboratory of Dr Chartier-Harlin from the convergence cohort (10 controls, 4 G2019S, 13 sporadic cases). Upon age- and sex-matching, a total of 13 controls, 7 G2019S LRRK2 PD and 13 sporadic PD samples were analysed. In this larger sampling, a significant centrosomal cohesion deficit was observed in lymphoblasts from G2019S LRRK2 PD as compared to healthy controls as well, and centrosomal alterations were reverted upon short-term application of 10 nM MLi2 (Figure 4A,B). Centrosomal deficits were observed in all G2019S LRRK2 PD samples as compared to control samples, and were reverted by MLi2 in all cases (Figure 4C). Importantly, a significant centrosomal cohesion deficit was also observed in a subset of sporadic PD patients, none of which were carriers of the G2019S LRRK2 mutation, as compared to healthy controls (Figures 4A and 4B). In three out of 13 sporadic patients, centrosomal cohesion deficits were similar to those observed in G2019S LRRK2-PD patients, and were reverted by short-term application of 10 nM MLi2 (Figures 4A and 4D).

### III. CONCLUSION

The objective of this study was to evaluate whether detecting centrosomal deficits may serve as target engagement biomarker and/or patient enrichment biomarker for LRRK2 inhibitor clinical trials. All G2019S LRRK2 LCLs analyzed displayed a centrosomal cohesion deficit without alterations in cell cycle or cell viability. Such cohesion phenotype was also observed in a subset of LCLs from sporadic PD patients, and was reverted in all cases upon kinase inhibitor treatment. Thus, this cellular readout may comprise a valid pharmacodynamic readout as well as patient enrichment biomarker of potential use in LRRK2 kinase inhibitor clinical trials. In addition, our data suggest that a subset of sporadic PD patients may also benefit from LRRK2-related treatment strategies, and that these patients can be identified using assays aimed at determining centrosomal alterations in cells derived from peripheral blood.

## Claims

1. A leucine-rich repeat kinase 2 (LRRK2) protein inhibitor for use in the treatment and/or prevention of Parkinson's disease (PD), or diseases related to PD, in a subject comprising centrosomal deficit.

2. A LRRK2 protein inhibitor for use according to claim 1, wherein
- the subject does not comprise the replacement of a Glycine (G) with a Serine (S) in the LRRK2 protein at the homologous position to position 2019 of sequence SEQ ID NO: 1 (G2019S), or
- the subject comprises the replacement of a Glycine (G) with a Serine (S) in the LRRK2 protein at the homologous position to position 2019 of sequence SEQ ID NO: 1 (G2019S) when the PD is an early stage of PD.

3. A LRRK2 protein inhibitor for use according to claim 1 or 2, wherein the subject comprises an increased expression levels of gene encoding the LRRK2 protein with respect to a reference level.

4. A LRRK2 protein inhibitor for use according to any one of claims 1 to 3, wherein the centrosomal deficit is centrosomal cohesion deficit.

5. LRRK2 protein inhibitor for use according to any one of claims 1 to 4, wherein the PD is sporadic PD (sPD) or familiar PD (fPD).

6. LRRK2 protein inhibitor for use according to any one of claims 1 to 5, wherein the diseases related to PD are Multiple System Atrophy, progressive supranuclear palsy and corticobasal degeneration, dementia with Lewy bodies or frontotemporal dementia.

7. LRRK2 protein inhibitor for use according to any one of claims 1 to 6, wherein the subject comprises at least one mutation in the LRRK2 protein different from G2019S, preferably, the mutation in the LRRK2 protein different from G2019S is the replacement of an Arginine (R) with a Cysteine (C) at homologous position to position 1441 of sequence SEQ ID NO: 1 (R1441C).

8. LRRK2 protein inhibitor for use according to any one of claims 1 to 7, wherein the LRRK2 protein inhibitor is selected from the group consisting of a chemical compound, a LRRK2 specific siRNA, a LRRK2 specific antisense oligonucleotide, a LRRK2 specific ribozyme and a LRRK2 specific antibody, preferably, the chemical compound is 3-(4-Pyrimidinyl) Indazole (MLi-2), a dihydrobenzothiophene, a 2-(benzyloxy)-5-(2-fluoropyridin-4-yl)-N-(pyridin-3-yl)benzamide (GSK2578215A), and derivatives thereof.

9. LRRK2 protein inhibitor according to any one of claims 1 to 8, wherein the inhibitor is comprised within a pharmaceutical composition and/or is administered in combination with another compound useful for treating PD.

10. An *in vitro* method for designing a customized therapy for a subject with PD, or diseases related to PD, comprising determining the presence of centrosomal deficit in a biological sample isolated from said subject, wherein if the subject comprises centrosomal deficit then the subject is susceptible to receive a therapy based on a LRRK2 protein inhibitor.

11. An *in vitro* method according to claim 10, wherein the centrosomal deficit is centrosomal cohesion deficit.

12. An *in vitro* method according to claim 10 or 11, wherein the method further comprises determining the presence of the replacement of a Glycine (G) with a Serine (S) in the LRRK2 protein at homologous position to position 2019 of sequence SEQ ID NO: 1 in said biological sample, wherein
- if the subject does not comprise the replacement of a Glycine (G) with a Serine (S) in the LRRK2 protein at the homologous position to position 2019 of sequence SEQ ID NO: 1, or
- if the subject comprises the replacement of a Glycine (G) with a Serine (S) in the LRRK2 protein at the homologous position to position 2019 of sequence SEQ ID NO: 1 when the PD is an early stage of PD,
then the subject is susceptible to receive a therapy based on a LRRK2 protein inhibitor.

13. An *in vitro* method according to any one of claims 10 to 12, wherein the method further comprises determining the expression levels of the gene encoding the LRRK2 protein, wherein if the expression levels of said gene are increased with respect to a reference level, then subject is susceptible to receive a therapy based on a LRRK2 protein inhibitor.

14. An *in vitro* method according to any one of claims 10 to 13, wherein the diseases related to PD are Multiple System Atrophy, progressive supranuclear palsy and corticobasal degeneration, dementia with Lewy bodies or frontotemporal dementia.

15. Method according to any one of claims 10 to 14, wherein the LRRK2 protein inhibitor is selected from the group consisting of a chemical compound, a LRRK2 specific siRNA, a LRRK2 specific antisense oligonucleotide, a LRRK2 specific ribozyme and a LRRK2 specific antibody, preferably, the chemical compound is 3-(4-Pyrimidinyl) Indazole (MLi-2), a dihydrobenzothiophene, a 2-(benzyloxy)-5-(2-fluoropyridin-4-yl)-N-(pyridin-3-yl)benzamide (GSK2578215A), and derivatives thereof.


SEQUENCE LISTING

<110>  CONSEJO SUPERIOR DE INVESTIGACIONES CIENTÍFICAS (CSIC)
       UNIVERSITÉ DE LILLE
       INSERM (INSTITUT NATIONAL DE LA SANTÉ ET DE LA RECHERCHE MÉDICALE)
       CENTRE HOSPITALIER REGIONAL UNIVERSITAIRE DE LILLE

<120>  Compounds useful for the treatment of Parkinson's disease

<130>  EP1641.1444

<160>  6

<170>  PatentIn version 3.5

<210>  1
<211>  2527
<212>  PRT
<213>  Homo sapiens

<400>  1

Met Ala Ser Gly Ser Cys Gln Gly Cys Glu Glu Asp Glu Glu Thr Leu
1               5                   10                  15

Lys Lys Leu Ile Val Arg Leu Asn Asn Val Gln Glu Gly Lys Gln Ile
            20                  25                  30

Glu Thr Leu Val Gln Ile Leu Glu Asp Leu Leu Val Phe Thr Tyr Ser
        35                  40                  45

Glu Arg Ala Ser Lys Leu Phe Gln Gly Lys Asn Ile His Val Pro Leu
    50                  55                  60

Leu Ile Val Leu Asp Ser Tyr Met Arg Val Ala Ser Val Gln Gln Val
65                  70                  75                  80

Gly Trp Ser Leu Leu Cys Lys Leu Ile Glu Val Cys Pro Gly Thr Met
                85                  90                  95

Gln Ser Leu Met Gly Pro Gln Asp Val Gly Asn Asp Trp Glu Val Leu
            100                 105                 110

Gly Val His Gln Leu Ile Leu Lys Met Leu Thr Val His Asn Ala Ser
        115                 120                 125

Val Asn Leu Ser Val Ile Gly Leu Lys Thr Leu Asp Leu Leu Leu Thr
    130                 135                 140

Ser Gly Lys Ile Thr Leu Leu Ile Leu Asp Glu Glu Ser Asp Ile Phe
145                 150                 155                 160

Met Leu Ile Phe Asp Ala Met His Ser Phe Pro Ala Asn Asp Glu Val

165                 170                 175

Gln Lys Leu Gly Cys Lys Ala Leu His Val Leu Phe Glu Arg Val Ser
        180                 185                 190

Glu Glu Gln Leu Thr Glu Phe Val Glu Asn Lys Asp Tyr Met Ile Leu
        195                 200                 205

Leu Ser Ala Leu Thr Asn Phe Lys Asp Glu Glu Glu Ile Val Leu His
    210                 215                 220

Val Leu His Cys Leu His Ser Leu Ala Ile Pro Cys Asn Asn Val Glu
225                 230                 235                 240

Val Leu Met Ser Gly Asn Val Arg Cys Tyr Asn Ile Val Val Glu Ala
                245                 250                 255

Met Lys Ala Phe Pro Met Ser Glu Arg Ile Gln Glu Val Ser Cys Cys
            260                 265                 270

Leu Leu His Arg Leu Thr Leu Gly Asn Phe Phe Asn Ile Leu Val Leu
        275                 280                 285

Asn Glu Val His Glu Phe Val Val Lys Ala Val Gln Gln Tyr Pro Glu
    290                 295                 300

Asn Ala Ala Leu Gln Ile Ser Ala Leu Ser Cys Leu Ala Leu Leu Thr
305                 310                 315                 320

Glu Thr Ile Phe Leu Asn Gln Asp Leu Glu Glu Lys Asn Glu Asn Gln
                325                 330                 335

Glu Asn Asp Asp Glu Gly Glu Glu Asp Lys Leu Phe Trp Leu Glu Ala
            340                 345                 350

Cys Tyr Lys Ala Leu Thr Trp His Arg Lys Asn Lys His Val Gln Glu
        355                 360                 365

Ala Ala Cys Trp Ala Leu Asn Asn Leu Leu Met Tyr Gln Asn Ser Leu
    370                 375                 380

His Glu Lys Ile Gly Asp Glu Asp Gly His Phe Pro Ala His Arg Glu
385                 390                 395                 400

Val Met Leu Ser Met Leu Met His Ser Ser Ser Lys Glu Val Phe Gln
                405                 410                 415

Ala Ser Ala Asn Ala Leu Ser Thr Leu Leu Glu Gln Asn Val Asn Phe
        420                 425                 430

Arg Lys Ile Leu Leu Ser Lys Gly Ile His Leu Asn Val Leu Glu Leu
    435                 440                 445

Met Gln Lys His Ile His Ser Pro Glu Val Ala Glu Ser Gly Cys Lys
    450                 455                 460

Met Leu Asn His Leu Phe Glu Gly Ser Asn Thr Ser Leu Asp Ile Met
465                 470                 475                 480

Ala Ala Val Val Pro Lys Ile Leu Thr Val Met Lys Arg His Glu Thr
                485                 490                 495

Ser Leu Pro Val Gln Leu Glu Ala Leu Arg Ala Ile Leu His Phe Ile
            500                 505                 510

Val Pro Gly Met Pro Glu Glu Ser Arg Glu Asp Thr Glu Phe His His
        515                 520                 525

Lys Leu Asn Met Val Lys Lys Gln Cys Phe Lys Asn Asp Ile His Lys
    530                 535                 540

Leu Val Leu Ala Ala Leu Asn Arg Phe Ile Gly Asn Pro Gly Ile Gln
545                 550                 555                 560

Lys Cys Gly Leu Lys Val Ile Ser Ser Ile Val His Phe Pro Asp Ala
                565                 570                 575

Leu Glu Met Leu Ser Leu Glu Gly Ala Met Asp Ser Val Leu His Thr
            580                 585                 590

Leu Gln Met Tyr Pro Asp Asp Gln Glu Ile Gln Cys Leu Gly Leu Ser
        595                 600                 605

Leu Ile Gly Tyr Leu Ile Thr Lys Lys Asn Val Phe Ile Gly Thr Gly
    610                 615                 620

His Leu Leu Ala Lys Ile Leu Val Ser Ser Leu Tyr Arg Phe Lys Asp
625                 630                 635                 640

Val Ala Glu Ile Gln Thr Lys Gly Phe Gln Thr Ile Leu Ala Ile Leu
                645                 650                 655

Lys Leu Ser Ala Ser Phe Ser Lys Leu Leu Val His His Ser Phe Asp
            660                 665                 670

Leu Val Ile Phe His Gln Met Ser Ser Asn Ile Met Glu Gln Lys Asp
        675                 680                 685

Gln Gln Phe Leu Asn Leu Cys Cys Lys Cys Phe Ala Lys Val Ala Met
    690                 695                 700

Asp Asp Tyr Leu Lys Asn Val Met Leu Glu Arg Ala Cys Asp Gln Asn
705                 710                 715                 720

Asn Ser Ile Met Val Glu Cys Leu Leu Leu Leu Gly Ala Asp Ala Asn
                725                 730                 735

Gln Ala Lys Glu Gly Ser Ser Leu Ile Cys Gln Val Cys Glu Lys Glu
            740                 745                 750

Ser Ser Pro Lys Leu Val Glu Leu Leu Leu Asn Ser Gly Ser Arg Glu
        755                 760                 765

Gln Asp Val Arg Lys Ala Leu Thr Ile Ser Ile Gly Lys Gly Asp Ser
    770                 775                 780

Gln Ile Ile Ser Leu Leu Leu Arg Arg Leu Ala Leu Asp Val Ala Asn
785                 790                 795                 800

Asn Ser Ile Cys Leu Gly Gly Phe Cys Ile Gly Lys Val Glu Pro Ser
                805                 810                 815

Trp Leu Gly Pro Leu Phe Pro Asp Lys Thr Ser Asn Leu Arg Lys Gln
            820                 825                 830

Thr Asn Ile Ala Ser Thr Leu Ala Arg Met Val Ile Arg Tyr Gln Met
        835                 840                 845

Lys Ser Ala Val Glu Glu Gly Thr Ala Ser Gly Ser Asp Gly Asn Phe
    850                 855                 860

Ser Glu Asp Val Leu Ser Lys Phe Asp Glu Trp Thr Phe Ile Pro Asp
865                 870                 875                 880

Ser Ser Met Asp Ser Val Phe Ala Gln Ser Asp Asp Leu Asp Ser Glu
                885                 890                 895

Gly Ser Glu Gly Ser Phe Leu Val Lys Lys Lys Ser Asn Ser Ile Ser
            900                 905                 910

Val Gly Glu Phe Tyr Arg Asp Ala Val Leu Gln Arg Cys Ser Pro Asn
        915                 920                 925

Leu Gln Arg His Ser Asn Ser Leu Gly Pro Ile Phe Asp His Glu Asp
    930                 935                 940

Leu Leu Lys Arg Lys Arg Lys Ile Leu Ser Ser Asp Asp Ser Leu Arg
945                 950                 955                 960

Ser Ser Lys Leu Gln Ser His Met Arg His Ser Asp Ser Ile Ser Ser
                965                 970                 975

Leu Ala Ser Glu Arg Glu Tyr Ile Thr Ser Leu Asp Leu Ser Ala Asn
            980                 985                 990

Glu Leu Arg Asp Ile Asp Ala Leu  Ser Gln Lys Cys Cys  Ile Ser Val
        995                 1000                 1005

His Leu  Glu His Leu Glu Lys  Leu Glu Leu His Gln  Asn Ala Leu
    1010                 1015                 1020

Thr Ser  Phe Pro Gln Gln Leu  Cys Glu Thr Leu Lys  Ser Leu Thr
    1025                 1030                 1035

His Leu  Asp Leu His Ser Asn  Lys Phe Thr Ser Phe  Pro Ser Tyr
    1040                 1045                 1050

Leu Leu  Lys Met Ser Cys Ile  Ala Asn Leu Asp Val  Ser Arg Asn
    1055                 1060                 1065

Asp Ile  Gly Pro Ser Val Val  Leu Asp Pro Thr Val  Lys Cys Pro
    1070                 1075                 1080

Thr Leu  Lys Gln Phe Asn Leu  Ser Tyr Asn Gln Leu  Ser Phe Val
    1085                 1090                 1095

Pro Glu  Asn Leu Thr Asp Val  Val Glu Lys Leu Glu  Gln Leu Ile
    1100                 1105                 1110

Leu Glu  Gly Asn Lys Ile Ser  Gly Ile Cys Ser Pro  Leu Arg Leu
    1115                 1120                 1125

Lys Glu  Leu Lys Ile Leu Asn  Leu Ser Lys Asn His  Ile Ser Ser
    1130                 1135                 1140

Leu Ser  Glu Asn Phe Leu Glu  Ala Cys Pro Lys Val  Glu Ser Phe
    1145                 1150                 1155

Ser Ala  Arg Met Asn Phe Leu  Ala Ala Met Pro Phe  Leu Pro Pro

1160                1165                1170

Ser Met Thr Ile Leu Lys Leu Ser Gln Asn Lys Phe Ser Cys Ile
    1175                1180                1185

Pro Glu Ala Ile Leu Asn Leu Pro His Leu Arg Ser Leu Asp Met
    1190                1195                1200

Ser Ser Asn Asp Ile Gln Tyr Leu Pro Gly Pro Ala His Trp Lys
    1205                1210                1215

Ser Leu Asn Leu Arg Glu Leu Leu Phe Ser His Asn Gln Ile Ser
    1220                1225                1230

Ile Leu Asp Leu Ser Glu Lys Ala Tyr Leu Trp Ser Arg Val Glu
    1235                1240                1245

Lys Leu His Leu Ser His Asn Lys Leu Lys Glu Ile Pro Pro Glu
    1250                1255                1260

Ile Gly Cys Leu Glu Asn Leu Thr Ser Leu Asp Val Ser Tyr Asn
    1265                1270                1275

Leu Glu Leu Arg Ser Phe Pro Asn Glu Met Gly Lys Leu Ser Lys
    1280                1285                1290

Ile Trp Asp Leu Pro Leu Asp Glu Leu His Leu Asn Phe Asp Phe
    1295                1300                1305

Lys His Ile Gly Cys Lys Ala Lys Asp Ile Ile Arg Phe Leu Gln
    1310                1315                1320

Gln Arg Leu Lys Lys Ala Val Pro Tyr Asn Arg Met Lys Leu Met
    1325                1330                1335

Ile Val Gly Asn Thr Gly Ser Gly Lys Thr Thr Leu Leu Gln Gln
    1340                1345                1350

Leu Met Lys Thr Lys Lys Ser Asp Leu Gly Met Gln Ser Ala Thr
    1355                1360                1365

Val Gly Ile Asp Val Lys Asp Trp Pro Ile Gln Ile Arg Asp Lys
    1370                1375                1380

Arg Lys Arg Asp Leu Val Leu Asn Val Trp Asp Phe Ala Gly Arg
    1385                1390                1395

Glu Glu Phe Tyr Ser Thr His Pro His Phe Met Thr Gln Arg Ala
    1400                1405                1410

Leu Tyr Leu Ala Val Tyr Asp Leu Ser Lys Gly Gln Ala Glu Val
    1415                1420                1425

Asp Ala Met Lys Pro Trp Leu Phe Asn Ile Lys Ala Arg Ala Ser
    1430                1435                1440

Ser Ser Pro Val Ile Leu Val Gly Thr His Leu Asp Val Ser Asp
    1445                1450                1455

Glu Lys Gln Arg Lys Ala Cys Met Ser Lys Ile Thr Lys Glu Leu
    1460                1465                1470

Leu Asn Lys Arg Gly Phe Pro Ala Ile Arg Asp Tyr His Phe Val
    1475                1480                1485

Asn Ala Thr Glu Glu Ser Asp Ala Leu Ala Lys Leu Arg Lys Thr
    1490                1495                1500

Ile Ile Asn Glu Ser Leu Asn Phe Lys Ile Arg Asp Gln Leu Val
    1505                1510                1515

Val Gly Gln Leu Ile Pro Asp Cys Tyr Val Glu Leu Glu Lys Ile
    1520                1525                1530

Ile Leu Ser Glu Arg Lys Asn Val Pro Ile Glu Phe Pro Val Ile
    1535                1540                1545

Asp Arg Lys Arg Leu Leu Gln Leu Val Arg Glu Asn Gln Leu Gln
    1550                1555                1560

Leu Asp Glu Asn Glu Leu Pro His Ala Val His Phe Leu Asn Glu
    1565                1570                1575

Ser Gly Val Leu Leu His Phe Gln Asp Pro Ala Leu Gln Leu Ser
    1580                1585                1590

Asp Leu Tyr Phe Val Glu Pro Lys Trp Leu Cys Lys Ile Met Ala
    1595                1600                1605

Gln Ile Leu Thr Val Lys Val Glu Gly Cys Pro Lys His Pro Lys
    1610                1615                1620

Gly Ile Ile Ser Arg Arg Asp Val Glu Lys Phe Leu Ser Lys Lys
    1625                1630                1635

Arg Lys Phe Pro Lys Asn Tyr Met Ser Gln Tyr Phe Lys Leu Leu
    1640                1645                1650

Glu Lys Phe Gln Ile Ala Leu Pro Ile Gly Glu Glu Tyr Leu Leu
    1655                1660                1665

Val Pro Ser Ser Leu Ser Asp His Arg Pro Val Ile Glu Leu Pro
    1670                1675                1680

His Cys Glu Asn Ser Glu Ile Ile Ile Arg Leu Tyr Glu Met Pro
    1685                1690                1695

Tyr Phe Pro Met Gly Phe Trp Ser Arg Leu Ile Asn Arg Leu Leu
    1700                1705                1710

Glu Ile Ser Pro Tyr Met Leu Ser Gly Arg Glu Arg Ala Leu Arg
    1715                1720                1725

Pro Asn Arg Met Tyr Trp Arg Gln Gly Ile Tyr Leu Asn Trp Ser
    1730                1735                1740

Pro Glu Ala Tyr Cys Leu Val Gly Ser Glu Val Leu Asp Asn His
    1745                1750                1755

Pro Glu Ser Phe Leu Lys Ile Thr Val Pro Ser Cys Arg Lys Gly
    1760                1765                1770

Cys Ile Leu Leu Gly Gln Val Val Asp His Ile Asp Ser Leu Met
    1775                1780                1785

Glu Glu Trp Phe Pro Gly Leu Leu Glu Ile Asp Ile Cys Gly Glu
    1790                1795                1800

Gly Glu Thr Leu Leu Lys Lys Trp Ala Leu Tyr Ser Phe Asn Asp
    1805                1810                1815

Gly Glu Glu His Gln Lys Ile Leu Leu Asp Asp Leu Met Lys Lys
    1820                1825                1830

Ala Glu Glu Gly Asp Leu Leu Val Asn Pro Asp Gln Pro Arg Leu
    1835                1840                1845

Thr Ile Pro Ile Ser Gln Ile Ala Pro Asp Leu Ile Leu Ala Asp
    1850                1855                1860

Leu Pro Arg Asn Ile Met Leu Asn Asn Asp Glu Leu Glu Phe Glu
    1865                1870                1875

Gln Ala Pro Glu Phe Leu Leu Gly Asp Gly Ser Phe Gly Ser Val
    1880                1885                1890

Tyr Arg Ala Ala Tyr Glu Gly Glu Glu Val Ala Val Lys Ile Phe
    1895                1900                1905

Asn Lys His Thr Ser Leu Arg Leu Leu Arg Gln Glu Leu Val Val
    1910                1915                1920

Leu Cys His Leu His His Pro Ser Leu Ile Ser Leu Leu Ala Ala
    1925                1930                1935

Gly Ile Arg Pro Arg Met Leu Val Met Glu Leu Ala Ser Lys Gly
    1940                1945                1950

Ser Leu Asp Arg Leu Leu Gln Gln Asp Lys Ala Ser Leu Thr Arg
    1955                1960                1965

Thr Leu Gln His Arg Ile Ala Leu His Val Ala Asp Gly Leu Arg
    1970                1975                1980

Tyr Leu His Ser Ala Met Ile Ile Tyr Arg Asp Leu Lys Pro His
    1985                1990                1995

Asn Val Leu Leu Phe Thr Leu Tyr Pro Asn Ala Ala Ile Ile Ala
    2000                2005                2010

Lys Ile Ala Asp Tyr Gly Ile Ala Gln Tyr Cys Cys Arg Met Gly
    2015                2020                2025

Ile Lys Thr Ser Glu Gly Thr Pro Gly Phe Arg Ala Pro Glu Val
    2030                2035                2040

Ala Arg Gly Asn Val Ile Tyr Asn Gln Gln Ala Asp Val Tyr Ser
    2045                2050                2055

Phe Gly Leu Leu Leu Tyr Asp Ile Leu Thr Thr Gly Gly Arg Ile
    2060                2065                2070

Val Glu Gly Leu Lys Phe Pro Asn Glu Phe Asp Glu Leu Glu Ile
    2075                2080                2085

Gln Gly Lys Leu Pro Asp Pro Val Lys Glu Tyr Gly Cys Ala Pro
    2090                2095                2100

Trp Pro Met Val Glu Lys Leu Ile Lys Gln Cys Leu Lys Glu Asn

2105                2110                2115

Pro Gln Glu Arg Pro Thr Ser Ala Gln Val Phe Asp Ile Leu Asn
    2120                2125                2130

Ser Ala Glu Leu Val Cys Leu Thr Arg Arg Ile Leu Leu Pro Lys
    2135                2140                2145

Asn Val Ile Val Glu Cys Met Val Ala Thr His His Asn Ser Arg
    2150                2155                2160

Asn Ala Ser Ile Trp Leu Gly Cys Gly His Thr Asp Arg Gly Gln
    2165                2170                2175

Leu Ser Phe Leu Asp Leu Asn Thr Glu Gly Tyr Thr Ser Glu Glu
    2180                2185                2190

Val Ala Asp Ser Arg Ile Leu Cys Leu Ala Leu Val His Leu Pro
    2195                2200                2205

Val Glu Lys Glu Ser Trp Ile Val Ser Gly Thr Gln Ser Gly Thr
    2210                2215                2220

Leu Leu Val Ile Asn Thr Glu Asp Gly Lys Lys Arg His Thr Leu
    2225                2230                2235

Glu Lys Met Thr Asp Ser Val Thr Cys Leu Tyr Cys Asn Ser Phe
    2240                2245                2250

Ser Lys Gln Ser Lys Gln Lys Asn Phe Leu Leu Val Gly Thr Ala
    2255                2260                2265

Asp Gly Lys Leu Ala Ile Phe Glu Asp Lys Thr Val Lys Leu Lys
    2270                2275                2280

Gly Ala Ala Pro Leu Lys Ile Leu Asn Ile Gly Asn Val Ser Thr
    2285                2290                2295

Pro Leu Met Cys Leu Ser Glu Ser Thr Asn Ser Thr Glu Arg Asn
    2300                2305                2310

Val Met Trp Gly Gly Cys Gly Thr Lys Ile Phe Ser Phe Ser Asn
    2315                2320                2325

Asp Phe Thr Ile Gln Lys Leu Ile Glu Thr Arg Thr Ser Gln Leu
    2330                2335                2340

Phe Ser Tyr Ala Ala Phe Ser Asp Ser Asn Ile Ile Thr Val Val
    2345                2350                2355

Val Asp Thr Ala Leu Tyr Ile Ala Lys Gln Asn Ser Pro Val Val
    2360                2365                2370

Glu Val Trp Asp Lys Lys Thr Glu Lys Leu Cys Gly Leu Ile Asp
    2375                2380                2385

Cys Val His Phe Leu Arg Glu Val Met Val Lys Glu Asn Lys Glu
    2390                2395                2400

Ser Lys His Lys Met Ser Tyr Ser Gly Arg Val Lys Thr Leu Cys
    2405                2410                2415

Leu Gln Lys Asn Thr Ala Leu Trp Ile Gly Thr Gly Gly Gly His
    2420                2425                2430

Ile Leu Leu Leu Asp Leu Ser Thr Arg Arg Leu Ile Arg Val Ile
    2435                2440                2445

Tyr Asn Phe Cys Asn Ser Val Arg Val Met Met Thr Ala Gln Leu
    2450                2455                2460

Gly Ser Leu Lys Asn Val Met Leu Val Leu Gly Tyr Asn Arg Lys
    2465                2470                2475

Asn Thr Glu Gly Thr Gln Lys Gln Lys Glu Ile Gln Ser Cys Leu
    2480                2485                2490

Thr Val Trp Asp Ile Asn Leu Pro His Glu Val Gln Asn Leu Glu
    2495                2500                2505

Lys His Ile Glu Val Arg Lys Glu Leu Ala Glu Lys Met Arg Arg
    2510                2515                2520

Thr Ser Val Glu
    2525

<210> 2
<211> 1271
<212> PRT
<213> Homo sapiens

<400> 2

Met Ala Ser Gly Ser Cys Gln Gly Cys Glu Glu Asp Glu Glu Thr Leu
1               5                   10                  15

Lys Lys Leu Ile Val Arg Leu Asn Asn Val Gln Glu Gly Lys Gln Ile
            20                  25                  30
Glu Thr Leu Val Gln Ile Leu Glu Asp Leu Leu Val Phe Thr Tyr Ser
        35                  40                  45
Glu Arg Ala Ser Lys Leu Phe Gln Gly Lys Asn Ile His Val Pro Leu
    50                  55                  60
Leu Ile Val Leu Asp Ser Tyr Met Arg Val Ala Ser Val Gln Gln Val
65                  70                  75                  80
Gly Trp Ser Leu Leu Cys Lys Leu Ile Glu Val Cys Pro Gly Thr Met
                85                  90                  95
Gln Ser Leu Met Gly Pro Gln Asp Val Gly Asn Asp Trp Glu Val Leu
            100                 105                 110
Gly Val His Gln Leu Ile Leu Lys Met Leu Thr Val His Asn Ala Ser
        115                 120                 125
Val Asn Leu Ser Val Ile Gly Leu Lys Thr Leu Asp Leu Leu Leu Thr
    130                 135                 140
Ser Gly Lys Ile Thr Leu Leu Ile Leu Asp Glu Glu Ser Asp Ile Phe
145                 150                 155                 160
Met Leu Ile Phe Asp Ala Met His Ser Phe Pro Ala Asn Asp Glu Val
                165                 170                 175
Gln Lys Leu Gly Cys Lys Ala Leu His Val Leu Phe Glu Arg Val Ser
            180                 185                 190
Glu Glu Gln Leu Thr Glu Phe Val Glu Asn Lys Asp Tyr Met Ile Leu
        195                 200                 205
Leu Ser Ala Leu Thr Asn Phe Lys Asp Glu Glu Glu Ile Val Leu His
    210                 215                 220
Val Leu His Cys Leu His Ser Leu Ala Ile Pro Cys Asn Asn Val Glu
225                 230                 235                 240
Val Leu Met Ser Gly Asn Val Arg Cys Tyr Asn Ile Val Val Glu Ala
                245                 250                 255
Met Lys Ala Phe Pro Met Ser Glu Arg Ile Gln Glu Val Ser Cys Cys
            260                 265                 270

Leu Leu His Arg Leu Thr Leu Gly Asn Phe Phe Asn Ile Leu Val Leu
        275                 280                 285

Asn Glu Val His Glu Phe Val Val Lys Ala Val Gln Gln Tyr Pro Glu
    290                 295                 300

Asn Ala Ala Leu Gln Ile Ser Ala Leu Ser Cys Leu Ala Leu Leu Thr
305                 310                 315                 320

Glu Thr Ile Phe Leu Asn Gln Asp Leu Glu Glu Lys Asn Glu Asn Gln
                325                 330                 335

Glu Asn Asp Asp Glu Gly Glu Glu Asp Lys Leu Phe Trp Leu Glu Ala
            340                 345                 350

Cys Tyr Lys Ala Leu Thr Trp His Arg Lys Asn Lys His Val Gln Glu
        355                 360                 365

Ala Ala Cys Trp Ala Leu Asn Asn Leu Leu Met Tyr Gln Asn Ser Leu
    370                 375                 380

His Glu Lys Ile Gly Asp Glu Asp Gly His Phe Pro Ala His Arg Glu
385                 390                 395                 400

Val Met Leu Ser Met Leu Met His Ser Ser Ser Lys Glu Val Phe Gln
                405                 410                 415

Ala Ser Ala Asn Ala Leu Ser Thr Leu Leu Glu Gln Asn Val Asn Phe
            420                 425                 430

Arg Lys Ile Leu Leu Ser Lys Gly Ile His Leu Asn Val Leu Glu Leu
        435                 440                 445

Met Gln Lys His Ile His Ser Pro Glu Val Ala Glu Ser Gly Cys Lys
    450                 455                 460

Met Leu Asn His Leu Phe Glu Gly Ser Asn Thr Ser Leu Asp Ile Met
465                 470                 475                 480

Ala Ala Val Val Pro Lys Ile Leu Thr Val Met Lys Arg His Glu Thr
                485                 490                 495

Ser Leu Pro Val Gln Leu Glu Ala Leu Arg Ala Ile Leu His Phe Ile
            500                 505                 510

Val Pro Gly Met Pro Glu Glu Ser Arg Glu Asp Thr Glu Phe His His
        515                 520                 525

Lys Leu Asn Met Val Lys Lys Gln Cys Phe Lys Asn Asp Ile His Lys
    530                 535                 540

Leu Val Leu Ala Ala Leu Asn Arg Phe Ile Gly Asn Pro Gly Ile Gln
545                 550                 555                 560

Lys Cys Gly Leu Lys Val Ile Ser Ser Ile Val His Phe Pro Asp Ala
                565                 570                 575

Leu Glu Met Leu Ser Leu Glu Gly Ala Met Asp Ser Val Leu His Thr
            580                 585                 590

Leu Gln Met Tyr Pro Asp Asp Gln Glu Ile Gln Cys Leu Gly Leu Ser
        595                 600                 605

Leu Ile Gly Tyr Leu Ile Thr Lys Lys Asn Val Phe Ile Gly Thr Gly
    610                 615                 620

His Leu Leu Ala Lys Ile Leu Val Ser Ser Leu Tyr Arg Phe Lys Asp
625                 630                 635                 640

Val Ala Glu Ile Gln Thr Lys Gly Phe Gln Thr Ile Leu Ala Ile Leu
                645                 650                 655

Lys Leu Ser Ala Ser Phe Ser Lys Leu Leu Val His His Ser Phe Asp
            660                 665                 670

Leu Val Ile Phe His Gln Met Ser Ser Asn Ile Met Glu Gln Lys Asp
        675                 680                 685

Gln Gln Phe Leu Asn Leu Cys Cys Lys Cys Phe Ala Lys Val Ala Met
    690                 695                 700

Asp Asp Tyr Leu Lys Asn Val Met Leu Glu Arg Ala Cys Asp Gln Asn
705                 710                 715                 720

Asn Ser Ile Met Val Glu Cys Leu Leu Leu Leu Gly Ala Asp Ala Asn
                725                 730                 735

Gln Ala Lys Glu Gly Ser Ser Leu Ile Cys Gln Val Cys Glu Lys Glu
            740                 745                 750

Ser Ser Pro Lys Leu Val Glu Leu Leu Leu Asn Ser Gly Ser Arg Glu
        755                 760                 765

Gln Asp Val Arg Lys Ala Leu Thr Ile Ser Ile Gly Lys Gly Asp Ser

770                 775                 780

Gln Ile Ile Ser Leu Leu Leu Arg Arg Leu Ala Leu Asp Val Ala Asn
785                 790                 795                 800

Asn Ser Ile Cys Leu Gly Gly Phe Cys Ile Gly Lys Val Glu Pro Ser
                805                 810                 815

Trp Leu Gly Pro Leu Phe Pro Asp Lys Thr Ser Asn Leu Arg Lys Gln
            820                 825                 830

Thr Asn Ile Ala Ser Thr Leu Ala Arg Met Val Ile Arg Tyr Gln Met
        835                 840                 845

Lys Ser Ala Val Glu Glu Gly Thr Ala Ser Gly Ser Asp Gly Asn Phe
    850                 855                 860

Ser Glu Asp Val Leu Ser Lys Phe Asp Glu Trp Thr Phe Ile Pro Asp
865                 870                 875                 880

Ser Ser Met Asp Ser Val Phe Ala Gln Ser Asp Asp Leu Asp Ser Glu
                885                 890                 895

Gly Ser Glu Gly Ser Phe Leu Val Lys Lys Lys Ser Asn Ser Ile Ser
            900                 905                 910

Val Gly Glu Phe Tyr Arg Asp Ala Val Leu Gln Arg Cys Ser Pro Asn
        915                 920                 925

Leu Gln Arg His Ser Asn Ser Leu Gly Pro Ile Phe Asp His Glu Asp
    930                 935                 940

Leu Leu Lys Arg Lys Arg Lys Ile Leu Ser Ser Asp Asp Ser Leu Arg
945                 950                 955                 960

Ser Ser Lys Leu Gln Ser His Met Arg His Ser Asp Ser Ile Ser Ser
                965                 970                 975

Leu Ala Ser Glu Arg Glu Tyr Ile Thr Ser Leu Asp Leu Ser Ala Asn
            980                 985                 990

Glu Leu Arg Asp Ile Asp Ala Leu  Ser Gln Lys Cys Cys  Ile Ser Val
        995                 1000                1005

His Leu  Glu His Leu Glu Lys  Leu Glu Leu His Gln  Asn Ala Leu
    1010                1015                1020

Thr Ser Phe Pro Gln Gln Leu Cys Glu Thr Leu Lys Ser Leu Thr
    1025                1030                1035

His Leu Asp Leu His Ser Asn Lys Phe Thr Ser Phe Pro Ser Tyr
    1040                1045                1050

Leu Leu Lys Met Ser Cys Ile Ala Asn Leu Asp Val Ser Arg Asn
    1055                1060                1065

Asp Ile Gly Pro Ser Val Val Leu Asp Pro Thr Val Lys Cys Pro
    1070                1075                1080

Thr Leu Lys Gln Phe Asn Leu Ser Tyr Asn Gln Leu Ser Phe Val
    1085                1090                1095

Pro Glu Asn Leu Thr Asp Val Val Glu Lys Leu Glu Gln Leu Ile
    1100                1105                1110

Leu Glu Gly Asn Lys Ile Ser Gly Ile Cys Ser Pro Leu Arg Leu
    1115                1120                1125

Lys Glu Leu Lys Ile Leu Asn Leu Ser Lys Asn His Ile Ser Ser
    1130                1135                1140

Leu Ser Glu Asn Phe Leu Glu Ala Cys Pro Lys Val Glu Ser Phe
    1145                1150                1155

Ser Ala Arg Met Asn Phe Leu Ala Ala Met Pro Phe Leu Pro Pro
    1160                1165                1170

Ser Met Thr Ile Leu Lys Leu Ser Gln Asn Lys Phe Ser Cys Ile
    1175                1180                1185

Pro Glu Ala Ile Leu Asn Leu Pro His Leu Arg Ser Leu Asp Met
    1190                1195                1200

Ser Ser Asn Asp Ile Gln Tyr Leu Pro Gly Pro Ala His Trp Lys
    1205                1210                1215

Ser Leu Asn Leu Arg Glu Leu Leu Phe Ser His Asn Gln Ile Ser
    1220                1225                1230

Ile Leu Asp Leu Ser Glu Lys Ala Tyr Leu Trp Ser Arg Val Glu
    1235                1240                1245

Lys Leu His Leu Ser His Asn Lys Leu Lys Glu Val Arg Arg Leu
    1250                1255                1260

Leu Pro Leu Lys Lys Tyr Thr Leu
    1265                1270

<210> 3
<211> 454
<212> PRT
<213> Homo sapiens

<220>
<221> misc_feature
<222> (1)..(1)
<223> Xaa can be any naturally occurring amino acid

<400> 3

Xaa Thr Gly Gly Arg Ile Val Glu Gly Leu Lys Phe Pro Asn Glu Phe
1               5                   10                  15

Asp Glu Leu Glu Ile Gln Gly Lys Leu Pro Asp Pro Val Lys Glu Tyr
            20                  25                  30

Gly Cys Ala Pro Trp Pro Met Val Glu Lys Leu Ile Lys Gln Cys Leu
        35                  40                  45

Lys Glu Asn Pro Gln Glu Arg Pro Thr Ser Ala Gln Val Phe Asp Ile
    50                  55                  60

Leu Asn Ser Ala Glu Leu Val Cys Leu Thr Arg Arg Ile Leu Leu Pro
65                  70                  75                  80

Lys Asn Val Ile Val Glu Cys Met Val Ala Thr His His Asn Ser Arg
                85                  90                  95

Asn Ala Ser Ile Trp Leu Gly Cys Gly His Thr Asp Arg Gly Gln Leu
            100                 105                 110

Ser Phe Leu Asp Leu Asn Thr Glu Gly Tyr Thr Ser Glu Glu Val Ala
        115                 120                 125

Asp Ser Arg Ile Leu Cys Leu Ala Leu Val His Leu Pro Val Glu Lys
    130                 135                 140

Glu Ser Trp Ile Val Ser Gly Thr Gln Ser Gly Thr Leu Leu Val Ile
145                 150                 155                 160

Asn Thr Glu Asp Gly Lys Lys Arg His Thr Leu Glu Lys Met Thr Asp
                165                 170                 175

Ser Val Thr Cys Leu Tyr Cys Asn Ser Phe Ser Lys Gln Ser Lys Gln

            180                 185                 190

Lys Asn Phe Leu Leu Val Gly Thr Ala Asp Gly Lys Leu Ala Ile Phe
        195                 200                 205

Glu Asp Lys Thr Val Lys Leu Lys Gly Ala Ala Pro Leu Lys Ile Leu
    210                 215                 220

Asn Ile Gly Asn Val Ser Thr Pro Leu Met Cys Leu Ser Glu Ser Thr
225                 230                 235                 240

Asn Ser Thr Glu Arg Asn Val Met Trp Gly Gly Cys Gly Thr Lys Ile
                245                 250                 255

Phe Ser Phe Ser Asn Asp Phe Thr Ile Gln Lys Leu Ile Glu Thr Arg
            260                 265                 270

Thr Ser Gln Leu Phe Ser Tyr Ala Ala Phe Ser Asp Ser Asn Ile Ile
        275                 280                 285

Thr Val Val Val Asp Thr Ala Leu Tyr Ile Ala Lys Gln Asn Ser Pro
    290                 295                 300

Val Val Glu Val Trp Asp Lys Lys Thr Glu Lys Leu Cys Gly Leu Ile
305                 310                 315                 320

Asp Cys Val His Phe Leu Arg Glu Val Met Val Lys Glu Asn Lys Glu
                325                 330                 335

Ser Lys His Lys Met Ser Tyr Ser Gly Arg Val Lys Thr Leu Cys Leu
            340                 345                 350

Gln Lys Asn Thr Ala Leu Trp Ile Gly Thr Gly Gly Gly His Ile Leu
        355                 360                 365

Leu Leu Asp Leu Ser Thr Arg Arg Leu Ile Arg Val Ile Tyr Asn Phe
    370                 375                 380

Cys Asn Ser Val Arg Val Met Met Thr Ala Gln Leu Gly Ser Leu Lys
385                 390                 395                 400

Asn Val Met Leu Val Leu Gly Tyr Asn Arg Lys Asn Thr Glu Glu Ile
                405                 410                 415

Gln Ser Cys Leu Thr Val Trp Asp Ile Asn Leu Pro His Glu Val Gln
            420                 425                 430

Asn Leu Glu Lys His Ile Glu Val Arg Lys Glu Leu Ala Glu Lys Met
        435                 440                 445

Arg Arg Thr Ser Val Glu
    450

<210>  4
<211>  521
<212>  PRT
<213>  Homo sapiens

<400>  4

Met Arg Val Ala Ser Val Gln Gln Val Gly Trp Ser Leu Leu Cys Lys
1               5                   10                  15

Leu Ile Glu Val Cys Pro Gly Thr Met Gln Ser Leu Met Gly Pro Gln
            20                  25                  30

Asp Val Gly Asn Asp Trp Glu Val Leu Gly Val His Gln Leu Ile Leu
        35                  40                  45

Lys Met Leu Thr Val His Asn Ala Ser Val Asn Leu Ser Val Ile Gly
    50                  55                  60

Leu Lys Thr Leu Asp Leu Leu Leu Thr Ser Gly Lys Ile Thr Leu Leu
65                  70                  75                  80

Ile Leu Asp Glu Glu Ser Asp Ile Phe Met Leu Ile Phe Asp Ala Met
                85                  90                  95

His Ser Phe Pro Ala Asn Asp Glu Val Gln Lys Leu Gly Cys Lys Ala
            100                 105                 110

Leu His Val Leu Phe Glu Arg Gly Asn Asn Val Glu Val Leu Met Ser
        115                 120                 125

Gly Asn Val Arg Cys Tyr Asn Ile Val Val Glu Ala Met Lys Ala Phe
    130                 135                 140

Pro Met Ser Glu Arg Ile Gln Glu Val Ser Cys Cys Leu Leu His Arg
145                 150                 155                 160

Leu Thr Leu Gly Asn Phe Phe Asn Ile Leu Val Leu Asn Glu Val His
                165                 170                 175

Glu Phe Val Val Lys Ala Val Gln Gln Tyr Pro Glu Asn Ala Ala Leu
            180                 185                 190

Gln Ile Ser Ala Leu Ser Cys Leu Ala Leu Leu Arg Ser Phe Pro Gly
        195                 200                 205

Ile Cys Glu Cys Ile Val Asn Ser Leu Arg Thr Lys Cys Asn Thr Ser
    210                 215                 220

Leu Asp Ile Met Ala Ala Val Val Pro Lys Ile Leu Thr Val Met Lys
225                 230                 235                 240

Arg His Glu Thr Ser Leu Pro Val Gln Leu Glu Ala Leu Arg Ala Ile
                245                 250                 255

Leu His Phe Ile Val Pro Gly Met Pro Glu Glu Ser Arg Glu Asp Thr
            260                 265                 270

Glu Phe His His Lys Leu Asn Met Val Lys Lys Gln Cys Phe Lys Asn
        275                 280                 285

Asp Ile His Lys Leu Val Leu Ala Ala Leu Asn Arg Phe Ile Gly Asn
    290                 295                 300

Pro Gly Ile Gln Lys Cys Gly Leu Lys Val Ile Ser Ser Ile Val His
305                 310                 315                 320

Phe Pro Asp Ala Leu Glu Met Leu Ser Leu Glu Gly Ala Met Asp Ser
                325                 330                 335

Val Leu His Thr Leu Gln Met Tyr Pro Asp Asp Gln Glu Ile Gln Cys
            340                 345                 350

Leu Gly Leu Ser Leu Ile Gly Tyr Leu Ile Thr Lys Lys Asn Val Phe
        355                 360                 365

Ile Gly Thr Gly His Leu Leu Ala Lys Ile Leu Val Ser Ser Leu Tyr
    370                 375                 380

Arg Phe Lys Asp Val Ala Glu Ile Gln Thr Lys Gly Phe Gln Thr Ile
385                 390                 395                 400

Leu Ala Ile Leu Lys Leu Ser Ala Ser Phe Ser Lys Leu Leu Val His
                405                 410                 415

His Ser Phe Asp Leu Val Ile Phe His Gln Met Ser Ser Asn Ile Met
            420                 425                 430

Glu Gln Lys Asp Gln Gln Phe Leu Asn Leu Cys Cys Lys Cys Phe Ala
        435                 440                 445

Lys Val Ala Met Asp Asp Tyr Leu Lys Asn Val Met Leu Glu Arg Ala
    450                 455                 460

Cys Asp Gln Asn Asn Ser Ile Met Val Glu Cys Leu Leu Leu Leu Gly
465                 470                 475                 480

Ala Asp Ala Asn Gln Ala Lys Glu Gly Ser Ser Leu Ile Cys Gln Val
                485                 490                 495

Cys Glu Lys Glu Ser Ser Pro Lys Leu Val Glu Leu Leu Leu Asn Ser
            500                 505                 510

Gly Ser Arg Glu Gln Asp Val Arg Lys
        515                 520

<210>  5
<211>  207
<212>  PRT
<213>  Homo sapiens

<220>
<221>  misc_feature
<222>  (1)..(1)
<223>  Xaa can be any naturally occurring amino acid

<400>  5

Xaa Leu Asp Leu Ser Ala Asn Glu Leu Arg Asp Ile Asp Ala Leu Ser
1               5                   10                  15

Gln Lys Cys Cys Ile Ser Val His Leu Glu His Leu Glu Lys Leu Glu
            20                  25                  30

Leu His Gln Asn Ala Leu Thr Ser Phe Pro Gln Gln Leu Cys Glu Thr
        35                  40                  45

Leu Lys Ser Leu Thr His Leu Asp Leu His Ser Asn Lys Phe Thr Ser
    50                  55                  60

Phe Pro Ser Tyr Leu Leu Lys Met Ser Cys Ile Ala Asn Leu Asp Val
65                  70                  75                  80

Ser Arg Asn Asp Ile Gly Pro Ser Val Val Leu Asp Pro Thr Val Lys
                85                  90                  95

Cys Pro Thr Leu Lys Gln Phe Asn Leu Ser Tyr Asn Gln Leu Ser Phe
            100                 105                 110

Val Pro Glu Asn Leu Thr Asp Val Val Glu Lys Leu Glu Gln Leu Ile

115                 120                 125

Leu Glu Gly Asn Lys Ile Ser Gly Ile Cys Ser Pro Leu Arg Leu Lys
    130                 135                 140

Glu Leu Lys Ile Leu Asn Leu Ser Lys Asn His Ile Ser Ser Leu Ser
145                 150                 155                 160

Glu Asn Phe Leu Glu Ala Cys Pro Lys Val Glu Ser Phe Ser Ala Arg
                165                 170                 175

Met Asn Phe Leu Val Ser Gly Cys Ser Val Val Pro Gln Gly Asn Leu
            180                 185                 190

Ile Gln Thr Val Glu Ser Gly Lys His Phe Cys Ala Cys Asp Ile
        195                 200                 205

<210>  6
<211>  78
<212>  PRT
<213>  Homo sapiens

<220>
<221>  misc_feature
<222>  (1)..(1)
<223>  Xaa can be any naturally occurring amino acid

<400>  6

Xaa Ile Val Arg Leu Asn Asn Val Gln Glu Gly Lys Gln Ile Glu Thr
1               5                   10                  15

Leu Val Gln Ile Leu Glu Asp Leu Leu Val Phe Thr Tyr Ser Glu Arg
            20                  25                  30

Ala Ser Lys Leu Phe Gln Gly Lys Asn Ile His Val Pro Leu Leu Ile
        35                  40                  45

Val Leu Asp Ser Tyr Met Arg Val Ala Ser Val Gln Gln Arg Trp Ser
    50                  55                  60

Leu Ala Met Leu Pro Arg Trp Val Gly His Phe Cys Ala Asn
65                  70                  75